Europäisches Patentamt

European Patent Office

Office européen des brevets

(11) Numéro de publication: **0 167 420**
A1

## (12) DEMANDE DE BREVET EUROPEEN

(21) Numéro de dépôt: 85400890.1

(22) Date de dépôt: 07.05.85

(51) Int. Cl.⁴: **C 12 N 15/00**
C 12 N 1/20, C 12 N 1/18
C 12 N 5/00, C 12 N 9/50
A 61 K 35/16

(30) Priorité: 09.05.84 FR 8407125

(43) Date de publication de la demande:
08.01.86 Bulletin 86/2

(84) Etats contractants désignés:
AT BE CH DE FR GB IT LI LU NL SE

(71) Demandeur: TRANSGENE S.A.
95 rue Saint-Lazare
F-75009 Paris(FR)

(72) Inventeur: Jaye, Michael
3017 South 2nd Street
Elkington 22204 Virginia(US)

(72) Inventeur: de la Salle, Henri
10, rue du Général Leclerc
F-67400 Ostwald(FR)

(72) Inventeur: Tolstoshev, Paul
5, rue Gounod
F-67450 Mundolsheim(FR)

(72) Inventeur: Lecocq, Jean-Pierre
6, rue du Champ du Feu
F-67116 Reichstett(FR)

(74) Mandataire: Warcoin, Jacques et al,
Cabinet Régimbeau 26, avenue Kléber
F-75116 Paris(FR)

(54) Vecteurs d'expression du facteur IX, cellules transformées par ces vecteurs et procédé de préparation du facteur IX.

(57) La présente invention concerne un vecteur de clonage et d'expression d'une protéine analogue au facteur IX dans des cellules, caractérisé en ce qu'il comporte au moins :

– une séquence d'ADN codant pour ladite protéine analogue au facteur IX : ADN.FIX ;

– les éléments assurant l'expression de cette séquence dans lesdites cellules.

Les cellules transformées par ces vecteurs sont utiles notamment pour la préparation du facteur IX.

EP 0 167 420 A1

Vecteurs d'expression du facteur IX, cellules transformées

par ces vecteurs et procédé de préparation du facteur IX.

La présente invention concerne la construction de lignées cellulaires produisant du facteur IX humain ou des molécules analogues.

Le facteur IX est une protéine intervenant au cours du cycle de la coagulation du sang. Il est synthétisé sous forme d'un zymogène et modifié post-traductionnellement par l'addition de chaine hydrocarbonée, l'hydroxylation d'un acide aspartique et la conversion des 12 acides glutamiques en acides $\gamma$-carboxy-glutamiques. Cette dernière modification dépend de la vitamine K (Bertina et coll., 1981).

Le foie est le site de synthèse du facteur IX.

Le facteur IX possède une activité réduite ou est absent dans le sang des hémophiles B. L'hémophilie B, comme la forme la plus fréquente de l'hémophilie, l'hémophilie A, se manifeste par un temps de saignement très prolongé. Elle est transmise sous forme d'un caractère récessif lié au sexe (Hedner et coll., 1982).

La nature moléculaire de la déficience en facteur IX est inconnue.

Le facteur IX lorsqu'il est fourni à des hémophiles B restaure des temps de saignement normaux.

Pour l'instant, la seule source disponible de facteur IX est le plasma humain, bien que dans certains cas la protéine bovine correspondante puisse être utilisée.

Les préparations de facteur IX peuvent être pyrogènes (provoquer des fièvres) et comportent aussi des risques de contamination des agents pathogènes ou des virus tels que le virus de l'hépatite et les agents vecteurs du S.I.D.A.

C'est pourquoi il est intéressant de développer un procédé permettant la préparation du facteur IX de très haute pureté. Le clonage moléculaire du cADN du facteur IX et son expression dans des cellules hôtes particulières sont des techniques appropriées pour obtenir des préparations de facteur IX exemptes des risques et des complications mentionnés précédemment.

La présente invention concerne le clonage du cADN du facteur IX et le développement de vecteurs plasmidiques autorisant l'expression du facteur IX dans les cellules hôtes suivantes :

1) Bactéries, en particulier Escherichia coli ;

2) Levures, en particulier Saccharomyces cerevisiae ;

3) Les cellules de mammifères.

Chaque type de cellules hôtes permet, par l'intermédiaire de la technologie des ADN recombinants, la synthèse de la protéine du facteur IX qui peut être reconnu immunologiquement par des anticorps spécifiques.

La présente invention concerne des vecteurs de clonage et d'expression de protéines analogues au facteur IX dans des cellules, caractérisé en ce qu'il comporte au moins :

- une séquence d'ADN codant pour ladite protéine analogue au facteur IX : ADN.FIX ;

- les éléments assurant l'expression de cette séquence dans lesdites cellules.

Ces vecteurs comportent, en outre, une origine de réplication dans lesdites cellules et codent pour un caractère de sélection positif s'exprimant dans lesdites cellules.

De façon générale, on entendra désigner dans la présente description par "protéine analogue au facteur IX" soit le facteur IX lui-même, soit une protéine ayant le même type d'activité biologique in vivo ou une activité apparentée, en particulier il peut s'agir d'une protéine ayant la même structure que le facteur IX à certains amino-acides près, ou bien des fragments du facteur IX ayant l'activité essentielle de la molécule complète.

La caractérisation plus fine des éléments du vecteur dépend bien entendu de la cellule hôte.

1) Cellules hôtes bactériennes

Dans le cas où les cellules hôtes sont des bactéries, par exemple Escherichia coli, les composants essentiels de ces vecteurs sont :

1) une origine de réplication dans les bactéries ;

2) un gène pour l'expression d'une résistance à un antibiotique qui assure une sélection positive des transformants ;

3) un promoteur qui peut être régulé, suivi par un site de fixation des ribosomes.

En aval du promoteur et du site de fixation des ribosomes, se trouve situé au moins un site de restriction  pour l'insertion d'un gène hétérologue, dans le cas présent le cADN codant pour le facteur IX ou un analogue. qui doit être exprimé sous le contrôle du promoteur régulable et du site de fixation des ribosomes.

La présence d'une origine de réplication pour le vecteur est essentielle pour permettre la réplication du vecteur dans les cellules bactériennes correspondantes, en particulier dans le cas de E. coli on utilisera, de préférence, l'origine de réplication du plasmide pBR322. Le plasmide pBR322 présente en effet l'avantage de donner un grand nombre de copies et ainsi d'augmenter la quantité de plasmides produisant la protéine désirée.

Parmi les promoteurs utilisables, on envisagera de préférence les promoteurs du bactériophage $\lambda$, en particulier le promoteur principal de gauche noté $\lambda P_L$. $P_L$ est un promoteur puissant responsable de la transcription précoce de $\lambda$.

Il est également possible d'utiliser d'autres promoteurs du bactériophage $\lambda$, notamment le promoteur de droite, $P_R$, ou le second promoteur de droite $P'_R$.

Bien qu'il soit possible d'utiliser des sites de fixation des ribosomes ou séquences d'initiation de la traduction très variés, on préfère utiliser celle de la protéine cII du bactériophage $\lambda$ qui sera nommée ci-après $\lambda$cIIrbs.

Comme cela sera démontré, il est également possible d'utiliser des séquences synthétiques, en particulier tout ou partie de la séquence :

ATAACACAGGAACAGATCTATG

Le vecteur en cause peut comporter, en outre, de préférence une fonction d'antiterminaison de transcription codée par exemple par le gène N de $\lambda$ noté $\lambda$N. En présence du produit de transcription du gène N la transcription à partir de $P_L$ se poursuit au delà de la plupart des signaux stop.

Ceci écarte les problèmes posés par un arrêt prématuré de la transcription qui peuvent se produire lorsque les gènes étrangers clonés présentent de tels signaux stop. En outre, il a été démontré que l'expression à partir de $P_L$ est améliorée dans un environnement $N^+$.

Toutefois, la présence de cette fonction n'est pas indispensable.

Afin d'écarter les problèmes de toxicité et d'instabilité du système hôte-vecteur en cas de production en continu de grandes quantités d'une protéine étrangère, il est nécessaire d'adjoindre un système controlant l'activité du promoteur

De préférence, le contrôle par la température de la synthèse de la protéine étrangère est effectué au niveau de la transcription au moyen d'un répresseur thermosensible codé dans la bactérie hôte, par exemple cI857 qui réprime l'activité de $P_L$ à 28°C mais qui est inactivé à 42°C. Le répresseur agit sur l'opérateur $O_L$ qui est adjacent au promoteur $P_L$. Bien que dans le cas précédent une partie du système d'expression thermo-inductible soit partie intégrante de la bactérie hôte, il est possible de prévoir que ce système fasse partie du vecteur lui-même.

Dans le cas du vecteur spécifique décrit ci-après et utilisé pour exprimer le facteur IX dans E. coli (pTG951), la résistance à l'antibiotique est obtenue par le gène de la β-lactamase qui confère à la bactérie une résistance à l'ampicilline ($\text{Amp}^r$) mais il est possible d'utiliser d'autres gènes de résistance.

Si de l'ampicilline se trouve dans le milieu de culture, seules les cellules exprimant le gène de la β-lactamase peuvent croître. De cette façon le présence d'une résistance à un antibiotique sur le vecteur d'expression permet la croissance des seules cellules portant ce plasmide.

Les exemples ci-après décriront la préparation de certains vecteurs, notamment de pTG320 qui assure l'expression du facteur IX dans E. coli.

La présente invention concerne en outre les bactéries, notamment les souches de E. coli, transformées par les vecteurs selon l'invention par des techniques connues et dont certaines seront rappelées dans les exemples.

Enfin l'invention concerne un procédé de préparation du facteur IX dans lequel on cultive sur un milieu de culture des bactéries transformées comme décrit précédemment et dans lequel on récupère ensuite le facteur IX formé.

Les milieux de culture mis en oeuvre sont connus de l'homme de métier et devront être adaptés à chaque souche cultivée. La culture sera de préférence effectuée en présence de l'antibiotique à l'encontre duquel la souche transformée est devenue résistante.

Le facteur IX est purifié après éclatement des cellules par des techniques connues comme la séparation sur colonne d'affinité ou la chromatographie d'exclusion.

La présente invention comporte, bien entendu, d'autres aspects, notamment les plasmides bactériens qui seront décrits dans les exemples ainsi que leurs mutants et dérivés et de façon générale les procédés de fermentation des bactéries transformées ainsi que le facteur IX ainsi obtenu.

## 2) Les levures

Les composants du vecteur d'expression du facteur IX dans des levures sont similaires à ceux utilisés pour les vecteurs d'expression dans E. coli.

Ces vecteurs comprennent :

1   une origine de réplication dans les levures ;

2)  un promoteur de levure qui peut être régulé et un terminateur de transcription ;

3)  un système de sélection susceptible d'être mise en oeuvre dans les levures ;

4)  une origine de réplication dans E. coli ;

5)  un gène pour l'expression d'une résistance à un antibiotique dans E. coli.

Le vecteur utilisé pour exprimer le facteur IX dans les levures (pTG834) contient deux origines de réplication. La première, celle de pBR322, autorise la réplication dans E. coli, tandis que la seconde, prise dans le plasmide 2 μ de levure, autorise la réplication plasmidique dans la levure.

La résistance à l'antibiotique est fournie par le gène de la β-lactamase qui confère une résistance à l'ampicilline et ainsi un marqueur sélectif dans E. coli.

Ce vecteur contient également le gène de levure URA3 qui complémente la mutation correspondante dans les levures ura3⁻. Sur milieu minimum, ceci sert comme système de sélection pour sélectionner les cellules portant les plasmides.

Le promoteur et le terminateur de transcription pour l'expression d'un gène hétérologue proviennent du gène de la phosphoglycérate-kinase de levure (PGK). Les gènes hétérologues qui doivent être exprimés sont clonés dans le site unique BglII qui est situé entre le promoteur et le terminateur de PGK.

3) Les cellules de mammifères

Dans le cas où les cellules hôtes sont des cellules animales, les éléments d'expression proviennent le plus fréquemment de génomes de virus, par exemple des virus de la famille des Papovaviridiae tels que SV40 (virus simien 40, polyomavirus) ou le BPV (virus du papillome bovin, papillomavirus). Mais on peut utiliser d'autres virus tels que des adenovirus.

Les éléments d'expression des vecteurs selon l'invention comprendront au moins un promoteur d'origine virale. Ainsi, dans les vecteurs dérivés de SV40 on peut utiliser le promoteur des gènes tardifs ou précoces, mais ce promoteur peut provenir d'un virus différent, ainsi dans le vecteur dérivé du BPV on utilisera le promoteur du gène de la thymidine kinase du virus Herpes simplex.

Afin d'améliorer l'expression, les vecteurs selon l'invention peuvent comprendre également des introns et des sites de polyadénylation situés dans cet ordre à l'extrémité de la séquence d'ADN clonée. Ces éléments peuvent, là aussi, provenir du virus vecteur ou bien être exogènes. Ainsi, dans le vecteur BPV les sites de polyadénylation proviennent de SV40. Parmi les introns utilisables, il faut citer l'intron I du gène de la β-globine de lapin, bien que cela ne soit nullement limitatif.

Enfin, un vecteur d'expression autonome doit être capable de se répliquer dans les cellules hôtes, le vecteur devra donc comporter une origine de réplication, par exemple origine de réplication de SV40 ou de BPV. En outre, le vecteur devra éventuellement être capable d'exprimer les protéines nécessaires à son maintien et à sa réplication dans lesdites cellules comme l'antigène T pour SV40.

Dans certains cas, les vecteurs comportant l'ensemble de ces éléments peuvent devenir trop importants pour être utilisables, dans ce cas certains des éléments nécessaires au maintien et à la réplication du vecteur peuvent être apportés par l'intermédiaire d'un autre vecteur ou bien être produits par les cellules elles-mêmes, en permettant de supprimer les parties correspondantes dans le vecteur.

Dans certains cas il peut être intéressant d'introduire dans le vecteur d'expression un marqueur, gène de sélection positive ou négative dans les cellules hôtes, ainsi par exemple un gène de résistance à un composé chimique particulier. Dans le cas de la construction BPV, ce gène est le gène codant pour la dhfr (dihydrofolate-réductase) qui confère aux cellules la résistance au méthotrexate.

Ce gène de sélection permet la manipulation des vecteurs dans les cellules hôtes et peut, dans certains cas, assurer leur maintien.

Les vecteurs ainsi obtenus sont utilisés pour transformer les cellules eucaryotes correspondantes selon des processus connus.

Dans le cas des cellules de mammifères, la transfection peut être effectuée par exemple par la méthode décrite par Wigler et coll., 1978 pour les ADN cellulaires, par des techniques de fusion de protoplastes ou de micro-injections, d'autres techniques sont connues lorsque le vecteur est sous forme virale.

Les cellules hôtes de mammifères peuvent être variées mais adaptées au vecteur mis en oeuvre.

Les cellules transformées ou transfectées sont cultivées sur un milieu approprié assurant leur croissance.

Après culture, les cellules sont récoltées et la protéine du facteur IX peut être isolée par des techniques connues dans le domaine de purification des protéines et des antigènes.

La présente invention concerne également les levures et les cellules de mammifère transformées, les procédés de culture de ces cellules ou levures et les facteurs IX ou analogues ainsi obtenus, ainsi d'ailleurs que les protéines analogues au facteur IX obtenues au moins en partie par culture de cellules bactériennes, de levures ou de mammifères.

D'autres caractéristiques et avantages de la présente invention seront mieux compris à la lecture des exemples ci-après et des dessins ci-annexés sur lesquels :
- la figure 1 représente le schéma de la sonde de facteur IX avec les modifications effectuées,
- la figure 2 représente la stratégie de séquençage de l'insert de cADN de FIX,
- la figure 3 représente le séquençage obtenu,

- la figure 4 représente la région codante et les 250 nucléotides de la région non codante 3' de pTG397,
- la figure 5 représente la stratégie de préparation de M13tg311,
- la figure 6 représente la stratégie de préparation de M13tg315,
- la figure 7 représente la stratégie de préparation de pTG907,
- la figure 8 représente la stratégie de préparation de M13tg910,
- la figure 9 représente la stratégie de préparation de pTG908,
- la figure 10 représente la stratégie de préparation de pTG909,
- la figure 11 représente la stratégie de préparation de pTG941,
- la figure 12 représente la stratégie de préparation de pTG320,
- la figure 13 représente la stratégie de préparation de pTG832
- la figure 14 représente la structure de pMOP,
- la figure 15 représente la stratégie de préparation de pTG326,

Les intitulés des figures suivantes sont simplifiés :

- la figure 16 est une photographie des immunoprécipités des produits de fermentation bactérienne,
- la figure 17 est une photographie des immunoprécipités des produits de fermentation de levure,
- la figure 18 est une photographie des immunoprécipités des produits de fermentation de culture cellulaire NIH 3T3,

- la figure 19 est identique à la figure 18 mais culture avec des concentration différentes de méthotrexate,
- la figure 20 est identique à la figure 15 mais avec immunoprécipitation avec un antisérum,
- la figure 21 est identique à la figure 18 mais culture de cellules LMTK avec une immunocompétition,
- la figure 22 est identique à la figure 18 mais incubation avec des précurseurs radioactifs.

Les exemples ci-après sont présentés de la façon suivante :

a) obtention d'un clone de cADN correspondant au facteur IX humain et clonage de ce cADN sur un phage,

b) clonage de ce cADN dans un vecteur bactérien,

c) clonage de ce cADN dans un vecteur de levure,

d) clonage de ce cADN dans un vecteur pour cellule de mammifère,

d) mise en évidence de l'expression du facteur IX grâce à ces différents vecteurs.

## Obtention d'un clone de cDNA correspondant
## au facteur IX humain

Pour obtenir un clone de cDNA correspondant au facteur IX humain, on utilise une sonde constituée d'un oligonucléotide synthétique unique. Compte tenu que seule la séquence d'acide aminé du facteur IX bovin est connue, une stratégie possible aurait pu être de composer un oligonucléotide permettant de trouver un clone de facteur IX bovin dans une banque de cDNA de foie de bovins. Ce clone de facteur IX bovin aurait pu être ensuite utilisé comme sonde dans une banque de cDNA de foie humain pour obtenir le clone humain correspondant.

De façon surprenante, il a été possible d'obtenir directement le clone du facteur IX à partir d'une banque de cDNA de foie humain en utilisant l'oligonucléotide synthétique, ce qui a écarté la nécessité d'effectuer une première sélection dans une banque de cDNA de foie de bovins. Cette stratégie est distincté de toutes celles qui ont été utilisées jusqu'à maintenant (Choo et coll., 1982 ; Karachi et coll., 1982) pour obtenir des clones de cDNA de facteur IX : nous avons utilisé une seule sonde oligonucléotide très longue, au lieu d'un mélange d'oligonucléotide plus court qui couvre tous les réarrangements possibles des codons.

Matériels et méthodes

1. Isolation de RNA messagers polyadénylés et synthèse du cDNA

Un foie fut obtenu post-mortem et rapidement refroidi dans l'azote liquide. Le RNA est préparé à partir de 5 g de foie en utilisant la technique d'extraction au chlorhydrate de guanidium 8 M, tel qu'il est décrit par Tolstoshev et coll., 1981.

14

Le RNA ainsi obtenu est chromatographié sur une colonne de polyU-Sepharose (Pharmacia) comme cela est indiqué par le fabricant de façon à obtenir un extrait enrichi en RNA contenant du poly-A. Les séquences de poly-A servent de guide pour la transcription réverse en utilisant des oligo (dT) comme "amorce", le cDNA a été synthétisé en utilisant 100 µL de réactif contenant 100 mM Tris-HCl, pH 8,3, 10 mM $MgCl_2$, 50 mM KCl, 30 mM - mercaptoéthanol, 10 µg/ml d'oligo (dT), 50 µg/ml poly-A-RNA, 0,5 mM de chaque de dATP, dGTP, dTTP, et dCTP, 80 unités de transcriptase reverse de virus de myoblaste avien (Life Sciences Inc.,). Après 45 minutes à 42°C, la réaction est terminée et le complexe de cDNA-RNA dénaturé par chauffage à 105°C pendant 3 minutes puis est transféré rapidement sur bain de glace.

Pour la synthèse du second brin de DNA, le mélange réactionnel précédent est dilué 5 fois et ajusté jusqu'à une concentration finale avec 100 mM HEPES-KOH, pH 6,9, 100 mM KCl, 200 µM de dATP, dGTP, dTTP, et P32-dCTP (activité spécifique 0,5 Ci/mmole). 10 unités de polymérase DNA de E. coli (fragment Klenow, (Boehringer Mannheim) sont ensuite ajoutées et l'incubation est effectuée à 25°C pendant 2 heures.

Le cDNA double brin (dscDNA) est extrait avec un volume égal de phénol : chloroforme (50 : 50) saturé avec 10 mM Tris-HCl pH 8,0, 1 mM EDTA et précipité deux fois à l'éthanol. Approximativement 970 ng de dsCDNA sont obtenus à partir de 5 µg de poly-A-RNA. Les extrémités sont rendues franches par digestion avec 5 unités de nucléase S1 dans un milieu réactionnel de 0,1 ml contenant 30 mM d'acétate de sodium, pH 4,8, 300 mM NaCl, 3 mM $ZnCl_2$. Après 1 heure à 37°C, l'EDTA et SDS sont ajoutés jusqu'à une concentration finale de 10 mM et 0,1 % respectivement et le mélange réactionnel est chauffé à 65°C pendant 5 minutes.

Le dscDNA digéré par S1 est alors appliqué sur un gradient de sucrose pré-établi 5-20 % contenant 100 mM Tris-HCl pH 7,5, 5 mM EDTA, 100 mM NaCl, et centrifugé à 30 000 tours par minutes à 15°C pendant 16 heures sur un rotor SW60 Ti. Les fractions de 0,5 ml sont collectées. Pour déterminer la taille du cDNA, un µl de chaque fraction est passé en électrophorèse sur un gel d'agarose neutre et la migration de chaque fraction est comparée avec un marqueur de poids moléculaire approprié. Les fractions contenant des cDNA plus grands que 1 kilobase sont rassemblées puis précipitées avec 2 volumes d'éthanol pendant une nuit en présence de 5 µg de tRNA de E. coli comme support. Le précipité est remis en suspension dans 20 µl 0,1 x SSC (5 mM NaCl, 0,5 mM de citrate de sodium pH 7,0).

## II. Clonage du cDNA

Des éléments d'extrémités homopolymères (approximativement 15 dCMP) sont ajoutés à l'extrémité 3' du cDNA en utilisant la transférase déoxynucléotidyl terminale(Deng et coll 1981.). De manière similaire des éléments d'extrémités homopolymères poly-dGMP (approximativement 10 dGMP) sont ajoutés aux extrémités 3' du plasmide pBR322 préalablement digéré avec l'enzyme de restriction PstI.

60 ng de cDNA possédant des extrémités de poly-CdCMP et 1 µg du vecteur préparé dans les conditions du paragraphe précédent sont chauffés pendant 2 heures à 42°C dans 10 mM Tris-HCl, pH 7,5, 100 mM NaCl. Les plasmides recombinants sont utilisés pour transformer la souche E. coli 8739 (Murray et coll. 1977) et les transformants sont sélectionnés sur plaque d'agar-LB contenant 15 µg/ml de tétracycline. On obtient 30 000 transformants dont environ 50 % comportent un insert de cDNA supérieur à 1 kilobase. Tous les transformants sont prélevés des plaques dans un volume minimum de LB et après addition d'un volume égal de glycérol, la banque obtenue est stockée à -20°C.

III. Sélection et synthèse de la sonde oligonucléotide pour le facteur IX

1 755 nucléotides de la séquence codant correspondant à la protéine bovine synthétisée par le foie sont analysées (voir Mac Gillivray et coll 1980 et Chung et coll 1981) de façon à mettre en évidence les codons utilisés préférentiellement.Nous avons sélectionné une séquence de 52 bases. Cet oligonucléotide est construit de la façon suivante :

Oligonucléotide A d(CTCACACTGATCACCATCCACATACT), B d(GCTTCCAGAACTCTGTAGTCTTCTCA), le fragment complémentaire C d(GGAAGCAGTATGT) sont synthétisés chimiquement par la méthode phosphotriester sur un support solide inorganique comme cela a été décrit précédemment par Kohli et coll 1982 et purifiés par HPLC comme décrit par Fritz 1978.

0,5 nmole d'oligonucléotide B sont mis en incubation pendant 30 minutes à 37°C dans un milieu réactionnel de 10 µl contenant 60 mM Tris-HCl pH 7,8, 6 mM $MgCL_2$, 6 mM dithiothreitol, 0,1 mM d'ATP, 6 pmole P32-ATP (3000 Ci/mmole), 2 unités de kinase T4 polynucléotide. L'oligomère B 5' phosphorylé est mélangé avec 0,5 nmole d'oligonucléotide A et C dans 10 mM Tris-HCl pH 7,5, 1 mM EDTA, chauffé à 100°C et le mélange est traité par refroidissement lent à 4°C. Le mélange réactionnel des oligonucléotides A et B est ligué dans 50 µl de mélange réactionnel contenant 66 mM Tris-HCl pH 7,5, 100 mM NaCl, 7,5 mM $MgCl_2$, 2 mM dithiothréitol, 0,5 mM spermidine, 0,2 mM EDTA, 4 unités de ligase DNA T4 à 4°C pendant une nuit. Le 52mer résultant est alors purifié du mélange de ligation par électrophorèse sur gel de polyacrylamide à 20 %.

IV. Analyse de la banque de cDNA de foie humain

10 000 colonies environ sont mises en croissance pendant une nuit sur une plaque d'agar LB contenant 10 µg/ml de tétracycline. Le jour suivant, les colonies bactériennes sont transférées sur filtres de nitrocellulose et les filtres sont préparés pour l'hybridation sur des colonies essentiellement comme cela a été décrit par Grunstein et coll. 1979. Les bactéries restantes, sur les plaques à la tétracycline d'origine, sont remises en croissance pendant quelques heures à 37°C.

La sonde d'hybridation est préparée par kination de 100 ng du 52mer avec 50 µc de P32-ATP. L'hybridation est effectuée à 48°C pendant une nuit dans 40 ml de 6 x SSC et 1 X de solution de Denhardt, 0,1 % SDS, 50 µg/ml d'E. coli tRNA. Le jour suivant les filtres sont lavés à 42°C plusieurs fois avec une solution de 6 x SSC, 0,1 % SDS. Les filtres sont séchés puis autoradiographiés pendant une nuit.

RESULTATS

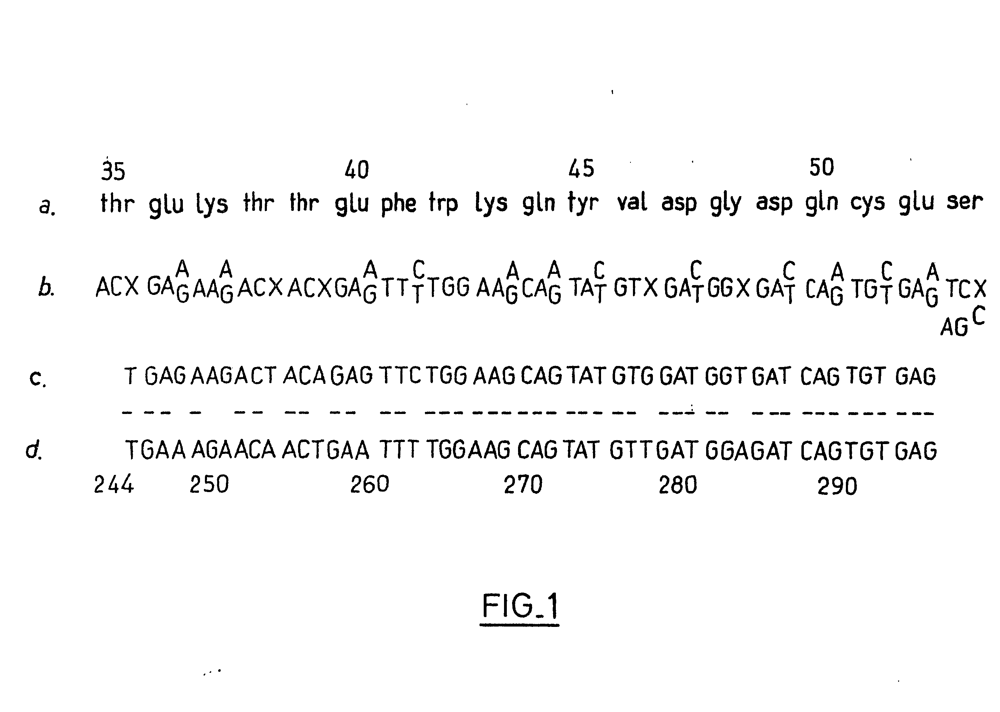

La séquence d'amino-acide du facteur IX bovin (voir Katayama et coll. 1979) est analysée pour trouver une séquence longue composée d'acides aminés déterminés par des codons faiblement dégénérés. La sonde a ainsi été réalisée pour correspondre aux acides aminés 36 à 52 du facteur IX bovin, qui correspond à 4 acides aminés codés par 4 codons (thr, val, gly), 12 acices aminés codés par 2 codons (glu, lys, phe, gln, tyr, asp, cys) et 1 acide aminé codé par 1 codon (trp) (figure 1).

Ensuite, l'analyse de 585 codons de la prothrombine bovine et du fibrinogène bovin ont révélé que le codon valine GTG est utilisé 21 fois sur 38. De même, TTC et TGT sont utilisés deux fois plus fréquemment que les autres codons dégénérés dans le cas des amino-acides phe et cys, respectivement.

Aucune autre préférence très nette ne se détache à l'analyse, toutefois, certains nucléotides sont trouvés peu fréquemment en 3ème position pour certains codons, par exemple G dans le cas de la glycine et de la thréonine.

En outre l'appariement G:T bien que moins stable que la paire de bases G:C contribuera au moins à la stabilisation de l'hybridation du 52mer durant l'hybridation de la colonie, tandis que l'appariement A:C ne le fera pas.

Ainsi G a été choisi pour chacun des acides aminés (glu, lys, gln) dans lesquels le choix en 3ème position du codon dégénéré était soit G soit A ; de même T a été choisi par rapport à A dans le cas des acides aminés tyr et asp. Compte tenu de ces considérations le choix s'est limité à T ou G comme nucléotide en 3ème position pour la thréonine et la glycine, toutefois comme cela a été indiqué précédemment, G se trouve peu fréquemment en 3ème position des codons de la thréonine. Ainsi on a choisi T pour l'un des codons de la thréonine et de façon à écarter un problème possible d'une structure secondaire à l'intérieur du 52mer, choisi T en position 3 pour la glycine et A en position 3 pour le second codon de la thréonine.

Sélection_de_la_banque_de_cDNA_humain_et_identification du_clone_de_facteur_IX

Lorsque l'on a eu réalisé la sonde 52mer, la stratégie d'origine était d'utiliser ce 52mer pour sélectionner la banque de cDNA de foie de bovin et ensuite utiliser ce clone de cDNA de facteur IX bovin sous forme d'une sonde pour une banque de cDNA de foie humain. Cette stratégie reposait sur une hypothèse de généralisation de l'homologie connue dans les séquences d'acides aminés à l'extrémité N des protéines bovines et humaines (voir Di Scipio et coll., 1977).

En s'appuyant sur cette homologie interspécifique dans les séquences d'amino-acides, on a effectué une sélection parallèle d'une banque de foie de bovin et de foie humain.

Les conditions d'hybridation et de lavage employées furent peu sévères car l'homologie actuelle entre la sonde 52mer et le cDNA de facteur IX étaient inconnus.

Différentes colonies dans la banque de cDNA bovin donnèrent des signaux positifs par sélection avec la sonde 52mer. Dans la banque de cDNA de foie humain, une colonie a été obtenue, qui donne un signal dans les conditions mises en oeuvre. Compte tenu du fait que la présente recherche a été effectuée dans le but d'isoler un cDNA de facteur IX humain, il n'a pas été effectué d'autres analyses sur les clones bovins et tous les efforts se sont portés sur le clone humain.

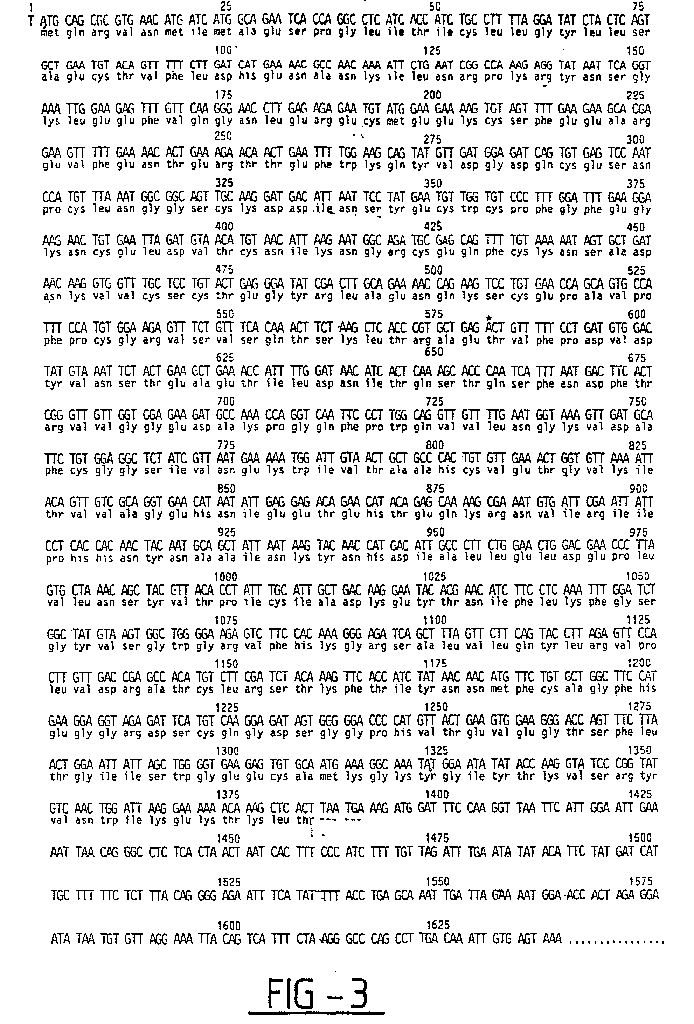

De façon à établir que cette colonie corresponde au facteur IX humain, le plasmide recombinant est digéré avec PstI et l'insert de cDNA excisé est transféré dans le phage M13mp8 (voir Messing et coll 1982) puis séquencé en utilisant des didéoxynucléotides comme terminateurs de chaîne (voir Sanger et coll 1977). La séquence obtenue correspondant aux nucléotides 1-180 de la figure 3. La séquence d'amino-acide déduite confirme que le clone est un clone de cDNA de facteur IX humain.

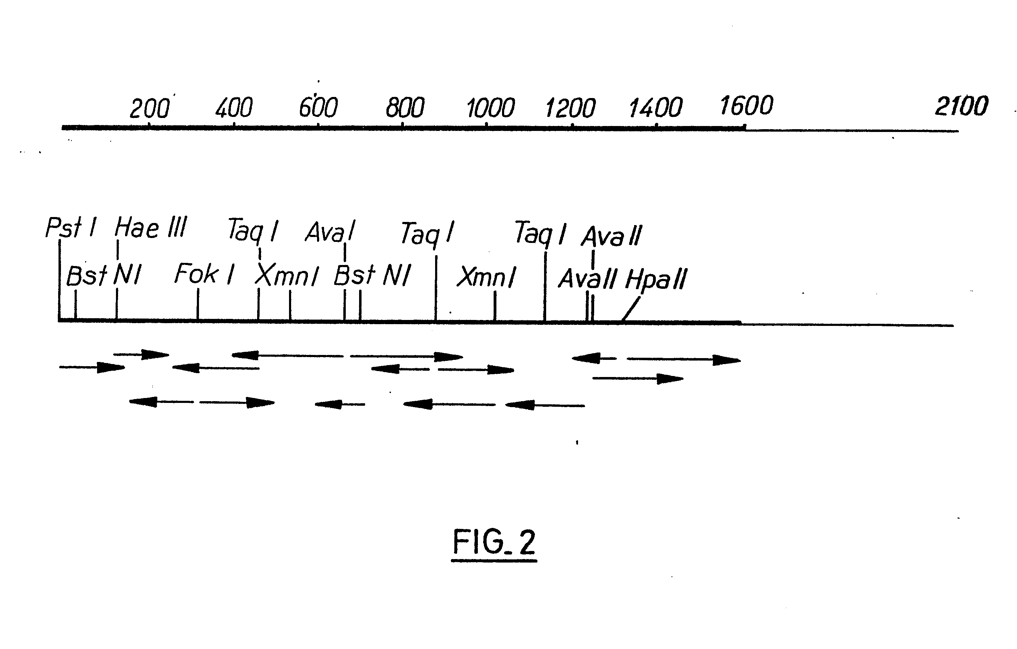

La séquence plus complète de l'insert de cDNA de ce clone (pTG397) est déterminée en combinant les méthodes de Maxam et de Gilbert, 1980, ou de Sanger, à partir de segments de restriction sous clonés dans M13mp9. La stratégie de séquençage est représentée dans la figure 2. La séquence obtenue est indiquée dans la figure 3.

DISCUSSION DU RESULTAT

En employant une sonde oligo-nucléotide unique, il a été possible d'isoler un clone de facteur IX humain contenant un insert de 2,1 kb. Lorsque l'insert de 2,1 kb est utilisé comme sonde pour trier une seconde banque de cDNA de foie humain, un autre clone spécifique de facteur IX (pTG398) contenant un insert de 2,6 kb est isolé.

L'extrémité 5' de pTG398 est situé au nucléotide 480 de la figure 3. Ceci suggère que l'extrémité non traduite 3' de pTG397 est incomplète et que l'extrémité complète 3' non traduite a une longueur d'environ 1,7 kb.

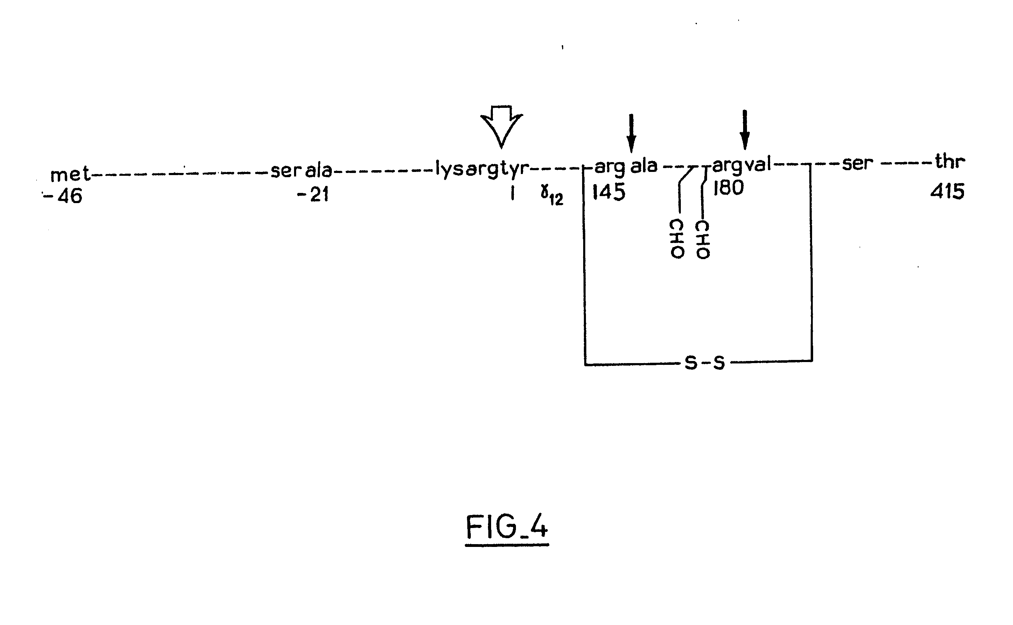

La séquence de la région codante entière et des 250 nucléotides de la région non codante 3' de pTG397 sont représentés dans la figure 4.

Plus de 75 % de cette séquence ont été établis sur les deux brins. On observe une différence entre la séquence de nucléotide obtenue et la séquence donnée par Kurachi et Davie. Cette différence comporte une transition au nucléotide 581 (G->A), transformant l'alanine en thréonine. La détermination de la séquence d'amino-acide du peptide de connection du facteur IX confirme la présence de thréonine dans cette position. Ces données suggèrent que comme pour d'autres protéines sériques le facteur IX est polymorphe (voir Cooper 1978).

En considérant l'utilisation des codons de substitution G:T et l'élimination de la structure secondaire possible dans la sonde de nucléotide, on a pu obtenir un 52mer unique qui s'est révélé être utile de façon satisfaisante comme sonde pour le séquencage d'un clone unique. Le 52mer choisi est horologue avec la séquence clonée pour 43/52 nucléotides tandis que 2 appariements erronés sont des paires G:T.

Ceci reflète une homologie à 85 % dans les séquences de nucléotides interspécifiques observées précédemment.

Structure_générale_du_cDNA_de_facteur_IX

La connaissance des séquences de nucléotide du cDNA de f.cteur IX permet de déduire la séquence d'amino-acide codée de la protéine correspondante. L'insert de cDNA de pTG397 révèle les caractéristiques suivantes du facteur IX humain :

a. Le facteur IX est une protéine contenant 415 acides aminés. Dans la figure 3, les nucléotides 140-1384 codent pour le facteur IX humain.

b. Le facteur IX humain est synthétisé sous forme d'un précurseur avec une séquence signal attaché et une séquence de préprotéine. Ceci est une caractéristique commune à toutes les protéines qui sont destinées à être sécrétées. Dans le cas du facteur IX, il y a une séquence signal classique d'environ 25 amino-acides codées par les nucléotides 2-76, inclus. L'extension hydrophobe des amino-acides est normalement éliminée pendant la sécrétion de la protéine à l'extérieur de la cellule. Les nucléotides 77-139 inclus codent pour une séquence de préprotéine qui probablement rapidement après la sécrétion est éliminée de la forme mature du facteur IX, (commençant avec la tyrosine au nucléotide 140).

Comme cela est souvent trouvé à la fin de la séquence de préprotéine, la structure d'amino-acides dibasiques lys-arg est trouvée à la jonction entre la séquence de préprotéine et le premier amino-acide du facteur IX mature.

c. La forme activée du facteur IX (IXa) est produite à partir de la forme inactive ou zymogène du facteur IX par hydrolyse de deux liaisons peptides par le facteur XI activé (XIa). Ces liaisons peptides sont trouvées entre les amino-acides arg-ala (nucléotides 572-577) et val-val (nucléotides 677-682). Le peptide le plus long de 35 amino-acides libéré pendant l'activation du facteur IX est appelé le peptide d'activation. Le facteur IXa est ainsi composé de deux chaînes polypeptidiques : une chaîne légère de 145 amino-acides (nucléotides 140-574) et une chaîne lourde de 235 amino-acides (nucléotides 680-1384). Les chaînes légères et lourdes du facteur IXa sont maintenues ensemble par des ponts disulfures. La chaîne lourde comprend un site actif typique de la sérine-protéase (met-phe-cys-ala-gly, nucléotides 1181-1195) de même que d'autres résidus importants dans l'activité catalytique de la sérine-protéase typique. La chaîne légère contient 12 unités d'acide glutamique qui sont $\gamma$-carboxylés pour produire l'acide $\gamma$-carboxy-glutamique par un processus qui dépend de la vitamine K (voir figure 4). Ceci est important dans la fixation du facteur IXa au $Ca^{++}$, à la membrane phospholipidique membranaire et au facteur VIIIa dans le cycle de coagulation.

d. Au contraire du facteur IX bovin qui a 4 sites pour la fixation d'hydrate de carbone, le facteur IX humain a 2 sites potentiels pour l'attachement des hydrates de carbone. Ces sites sont tous situés dans le peptide d'activation, aux nucléotides 608-616 et 638-646, et contiennent la séquence typique asn-x-thr/ser. Par contraste le facteur IX bovin a 4 hydrates de carbone attachés dont la structure a été récemment déterminée par Mizurochi et coll (1983).

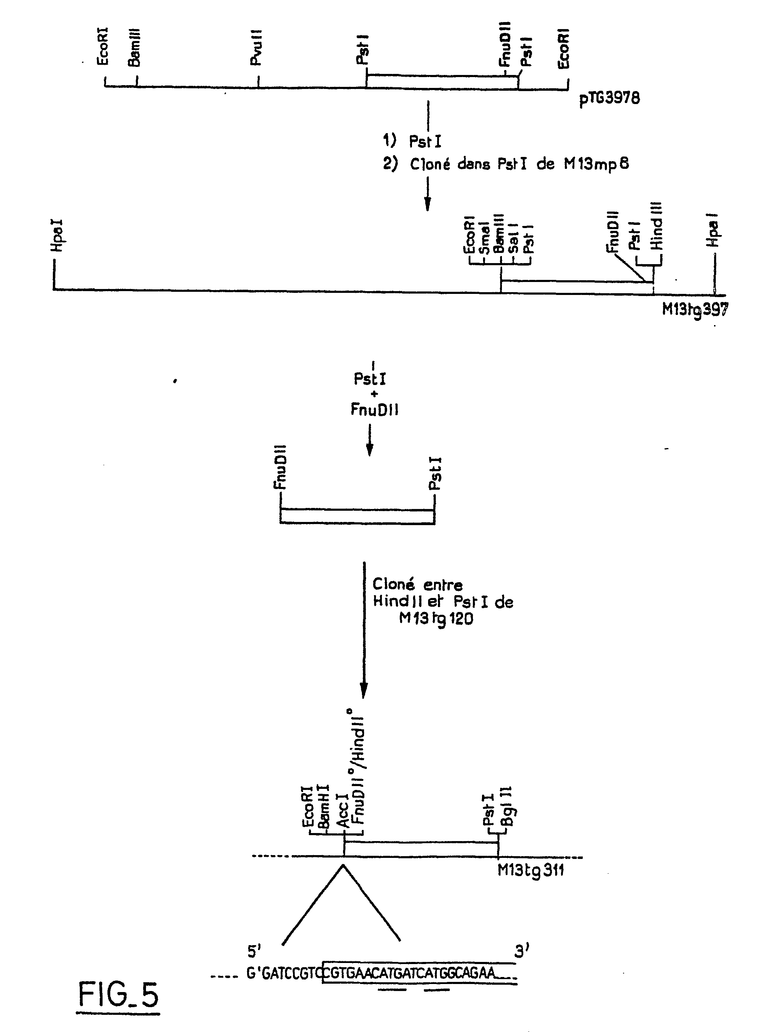

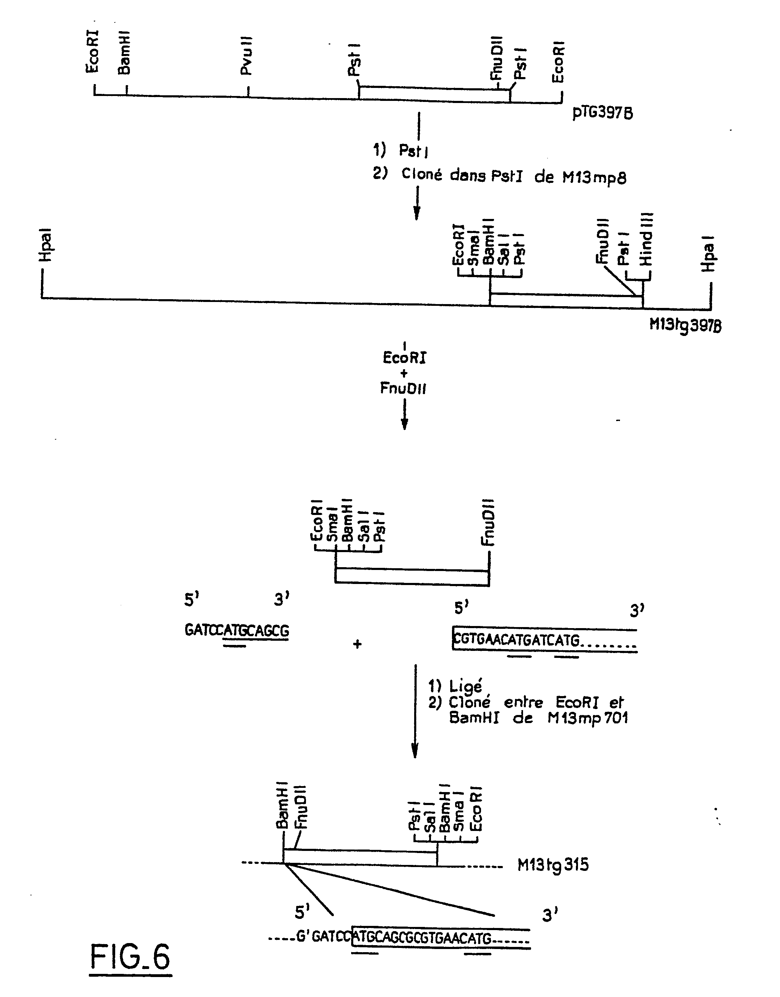

## PREPARATION DU cDNA DE FACTEUR IX CLONÉ NECESSAIRE POUR L'EXPRESSION (Voir figures 5 et 6).

pTG397 a été mis en digestion avec l'enzyme de restriction PstI. Comme la banque de cDNA a été clonée par des extrémités G/C dans le site PstI de pBR322 et en outre que le facteur IX de pTG397 ne contient pas un site interne de reconnaissance PstI, ce traitement libère un cDNA intact de 2,1 kb correspondant au facteur IX, avec des extrémités susceptibles d'être clonées dans les sites PstI. Le fragment de cDNA facteur IX est transféré par des procédures standards dans le phage M13MP8 qui a été précédemment mis en digestion avec PstI et traité avec la phosphatase alcaline. Les phages recombinants sont utilisés pour transfecter une souche de E. coli JM103.

Le phage recombinant M13tg397B est utilisé comme source de cDNA de FIX comme cela sera décrit ci-après.

Le cDNA de facteur IX contient un site de reconnaissance unique pour l'enzyme FnuDII qui reconnait la séquence de tétranucléotide CGCG située sur les nucléotides 7-10. Par digestion de M13tg397B avec PstI et FnuDII on obtient un grand fragment et de nombreux petits fragments, correspondants à 19 sites de reconnaissance pour FnuDII dans M13MP8. Le fragment le plus grand correspondant au fragment FnuDII:PstL.

Ce fragment écarte 9 nucléotides et les extrémités G/C de l'extrémité 5' du cDNA de facteur IX. Il a été considéré comme important d'écarter les extrémités G/C de l'extrémité 5' de façon à augmenter l'expression et la stabilité du cDNA de facteur IX dans le vecteur d'expression. En éliminant ces 9 nucléotides, on élimine un ATG de la séquence signal. Il n'est pas sûr toutefois que cet ATG, soit l'ATG qui initie la traduction. La portion du facteur IX éliminée par digestion avec FnuDII est dans la séquence signal et en conséquence n'est pas important pour l'activité du facteur IX.

Pour obtenir un site d'enzyme de restriction satisfaisant de part et d'autre du fragment FnuDII/PstI de facteur IX, ce fragment est cloné dans le phage M13tg120 préalablement digéré par HindII et PstI. Après transformation dans la cellule compétente JM103, les plages de recombinant blanches sont identifiées comme cela a été décrit précédemment.

Des répliquants de M13 sont préparés à partir des plaques blanches et l'analyse des séquences de nucléotides du phage recombinant M13tg311 confirme que la construction correcte a été obtenue.

M13tg311 permet l'excision du cDNA de facteur IX en utilisant une combinaison d'enzymes EcoRI et BglII ou par combinaison de BamHI plus BglII. La séquence de nucléotide à l'extrémité 5' du fragment excisé avec BamHI plus Bgl$_{II}$ est la suivante :

```
           1         10        20        30
5'    G GATCCGTCCGTGAACATGATCATGGCAGAATCACCAGGC........
      /             ----------===---===----------------------->
    BamHI

    CTGCA G........A GATCT
         /           /
        /           /
     PstI        BglII
```

La portion soulignée de cette séquence représente la portion du clone de cDNA de facteur IX provenant du fragment FnuDII/PstI de M13tg397B. Le reste du clone provient du vecteur M13tg120. La portion doublement soulignée de cette séquence indique la localisation des séquences ATG. Il s'agit de sites potentiels pour l'initiation de la traduction du mRNA de facteur IX.

Le fragment BamHI/BglII qui peut être facilement excisé à partir de M13tg311 peut être cloné dans deux types de vecteurs d'expression. Le premier type de vecteur contient un signal pour la transcription correcte, un site de fixation des ribosomes et un site d'initiation ATG. Dans ce vecteur, le facteur IX sera exprimé sous forme d'une protéine fusionnée, car la traduction commence sur un promoteur en amont et se continue sur 15 nucléotides précédant le premier codon ATG dans la séquence du facteur IX. Ceci conduira au moins à 5 amino-acides fusionnés sur l'extrémité de la séquence signal du facteur IX. .

26

**0167420**

Le second type de vecteur comportera un signal pour la transcription correcte, et un site de fixation des ribosomes si nécessaire. Dans ce vecteur la traduction du mRNA de facteur IX commencera au premier ou peut-être au second ATG indiqués dans la séquence précédente. Ces deux types de vecteurs comporteront une origine de réplication et un moyen de sélection (résistance à un antibiotique ou complémentation auxotrophique) dans les cellules hôtes appropriées.

De façon que les cellules n'expriment pas seulement le facteur IX mais puisse le sécréter, la séquence signal doit être relativement intacte pour ce qui concerne la présence et la distribution des fragments d'amino-acides hydrophobes et hydrophiles. Comme cela a été indiqué précédemment, la digestion de M13tg397B avec FnuDII élimine les nucléotides 1-9 de la séquence signal du facteur IX.

Compte tenu du fait que l'ATG le plus proche auquel peut être effectuée la traduction dans les vecteurs du second type décrits précédemment se trouve situé aux nucléotides 17-19, ceci signifie que la séquence signal (si la traduction débute normalement à l'ATG éliminé lors de la digestion par FnuDII, a été écourté de 5 amino-acides. De façon à déterminer si ces 5 amino-acides additionnels sont importants pour la sécrétion et/ou pour l'utilisation du facteur IX, la séquence de nucléotide éliminée par digestion avec FnuDII est restaurée en utilisant 2 oligonucléotides synthétiques :

A)    5'    GATCCATGCAGCG    3'
              --------

B)    5'    CGCTGCATG    3'

La séquence soulignée dans l'oligonucléotide A correspondant à la séquence écartée, moins le premier nucléotide T, du phage M13tg397B par digestion avec FnuDII. L'oligonucléotide B est complémentaire de l'oligonucléotide A. L'hybridation des oligonucléotides A et B donne la structure à double brins suivante qui possède des extrémités BamHI cohésives :

```
5'      GATCCATGCAGCG      3'
3'           GTACGTCGC     5'
```

Les oligonucléotides A et B sont ligués avec le fragment de cDNA de facteur IX EcoRI/FnuDII qui a été purifié à partir de M13tg397B. Le phage M13MP701 traité par la phosphatase-alkaline et soumis à la restriction par BamHI est alors ajouté pour la ligation et la ligation est effectuée pendant toute une nuit. Un aliquote du mélange de ligation final est transformé dans une cellule JM103. La séquence de nucléotide du phage recombinant M13tg315 qui a donné une plage blanche confirme que la construction est correcte. La digestion de M13tg315 avec BamHI libère un fragment de cDNA de facteur IX dont la séquence 5' est la suivante :

```
       1      10       20
5'     G GATCCATGCAGCGCGTGAACATG////////FIX///////////G GATCC 3'
      /  ...............                                /
      /       ----------------                         /
   BamHI                                            BamHI
```

La séquence correspondant à l'oligonucléotide A a été indiquée ci-dessus en traits pointillés. La séquence soulignée correspondant aux 15 nucléotides qui codent pour les 5 amino-acides absents de la séquence signal du fragment de cDNA de facteur IX BamHI/BglII de M13tg311.

Ces vecteurs M13tg311 et M13tg315 vont servir de source de cADN du facteur IX dans les expériences de clonage ci-après :

Il convient de remarquer que les différentes séquences de nucléotides figurant dans les dessins doivent être considérées comme faisant explicitement partie de la présente description, ces séquences n'ont pas été reproduites dans le corps du texte afin de ne pas l'alourdir inutilement.

1, Procédés généraux

a) Souches bactériennes

Les souches bactériennes utilisées dans le cadre de la présente invention sont les suivantes :

. TGE900 qui est une souche de E. coli ayant les caractéristiques suivantes : su⁻F⁻his ilv bio ($\lambda$cI857$\Delta$BamHI).

. N6437, souche de E. coli ayant les caractéristiques suivantes : F⁻his ilv gal⁺ $\Delta$8proC⁺ : tn10 lacIm15 ($\lambda$cI857$\Delta$BamHI).

. JM103 qui est une souche de E. coli ayant les caractéristiques suivantes : $\Delta$(lac-pro) sup$^E$ thi endA sbcB15 sttA rK⁻nK⁺ / F¹ traD36 proAB⁻ laci$^a$ lacZ$\Delta$m15.

b) Souches de levures

Saccharomyces cereviseae

segregeants TGY1sp1

            TGY2sp4

toutes deux ura3⁻, his 3⁻.

TGY1sp1 est un segregeant obtenu par croisement de la souche GFR18 (Mat$\alpha$ His3-11-15 Leu2-3-12) (FINK et coll.) avec la souche TGY10-1 (Mat Ura3-251-373-328). La souche TGY10-1 est un dérivé isogénique de la souche FL100 (ATCC 2283).

Les souches mentionnées précédemment ont été utilisées parce qu'elles étaient disponibles, mais il est bien entendu qu'il est possible d'utiliser d'autres souches dans la mesure où elles présentent certaines caractéristiques essentielles qui sont rappelées dans le cours de la description détaillée.

b) Préparation_des_ADN

Des mini-préparations d'ADN de plasmides ou de phages M13 sont effectuées comme cela est décrit par Ish-Horowitz (1981)        à la seule différence que l'ADN est précipité une seconde fois avec de l'éthanol avant d'être utilisé.

Les maxi-préparations sont effectuées comme cela est décrit dans la publication précédente, avec une purification complémentaire par un gradient de densité CaCl/bromure d'éthidium.

c) Techniques_de_clonages

Le traitement des ADN avec des enzymes de restriction est effectué, sauf indications contraires, en utilisant les conditions indiquées par le fabricant (New England Biolabs, Bethesda Research Laboratories et Boehringer Mannheim).

Les oligonucléotides utilisés proviennent soit de New England Biolab, Hana Biologics et Worthington Biochemical, ou sont synthétisées par les techniques Kohli et coll.

Tous les produits radioactifs ont été obtenus chez  Amersham International.

Lorsque cela est nécessaire, les phosphates des extrémités 5' sont éliminés en utilisant, soit une phosphatase alcaline bactérienne, soit une phosphatase d'intestin de veau, pendant 30 minutes à 37°C dans le tampon d'enzyme de restriction.

La réparation des extrémités cohésives utilisant la polymérase de Klenow (Boehringer Mannheim) est effectuée à 25°C pendant 15 minutes dans un mélange de 50 mMol. Tris HCl, pH 7,8, 5 mMol. $MgCl_2$, 10 mMol. de β-mercaptoéthanol, 0,4 mMol. de dNTPs avec l'enzyme et 10 à 200 µg/ml d'ADN.

La nucléase $S_1$ (Miles) est utilisée à 2 u/µg d'ADN à 25°C pendant 30 minutes dans un milieu 0,3 Mol. NaCl, 0,03 Mol. NaOAc, pH 4,8, 0,003 Mol. $ZnCl_2$.

Bal31 est utilisée selon le procédé de Panayotatos et coll. (1981). Les ligations sont effectuées à 15°C (sauf lorsque cela est indiqué différemment) pendant 4 à 24 heures en utilisant de l'ADN ligase $T_4$ (Boehringer Mannheim) avec 100 mMol. de NaCl, 66 mMol. de Tris HCl, pH 7,5, 10 mMol. de $MgCl_2$, 0,5 mMol. de spermidine, 0,2 mMol. de EDTA, 2 mMol. de DTT, 1 mMol. d'ATP, 0,1 mg/ml de BSA et 5 à 50 µg/ml d'ADN.

Pour la ligation d'extrémités cohésives, on utilise environ 30 unités/ml de ligase. Pour la ligation des extrémités franches on utilise environ 100 unités/ml de ligase.

Après chaque traitement enzymatique, l'ADN est extrait avec le phénol, deux fois avec octanol - chloroforme (1:8) puis précipitation avec l'éthanol. Dans le cas de petits fragments ( 100bp) une triple extraction avec le diéthyléther remplace la précipitation à l'éthanol.

Lorsque cela est nécessaire, du tARN de E. coli ou de levure est utilisé comme entraineur. Les adaptateurs moléculaires (Collaborative Research, Bethesda Research Laboratories, New England Biolabs) sont préhybridés et utilisés en excès molaire de 10 à 50 fois pour les extrémités franches d'ADN utilisant les conditions de tampon décrites précédemment avec 100 unités/ml de ligase $T_4$ à 4°C pendant 15 heures.

L'insertion des "linkers" non phosphorylés a été effectuée de la façon suivante :

Les linkers sont repris dans du tampon TE (10mM Tris.HCl pH 7,5, 1 mM $Na_2$EDTA) à la concentration de 1 mg/ml et prehybridés par refroidissement de 65°C à 4°C. La ligation est effectuée dans un tampon de concentration 30 mM NaCl, 30 mM Tris.HCl pH 7,5, 1mM spermidine, 0,25 mM ATP, 2 mM DTT, 0,2 mM $Na_2$EDTA et 0,1 mg/ml serum albumine bovine. En général, la ligation (10 à 50 µl est effectuée avec un excès molaire du linker sur les extrémités d'ADN de 80 fois, et une concentration d'ADN cible de 0,1 µM. La ligase est utilisée à la concentration de 100 à 200 unités/ml et la ligation est effectuée à 4°C pendant 16 heures. Les linkers excédentaires sont éliminés par précipitation à la spermine (Hoopes et Mc Clure, 1981). L'ADN est ensuite repris dans 10 mM Tris.HCl pH 7,5, 1 mM $Na_2$EDTA, puis dilué dans du tampon d'hybridation (100 mM Tris.HCl pH 7,5, 5 mM $Na_2$EDTA, 100 mM NaCl). La réhybridation est effectué comme la préhybidation des linkers, et des aliquots sont transformés directement dans E. coli. Le principe de la méthode est décrit par LATHE et Coll., 1983.

Lorsque l'on utilise des adaptateurs phosphorylés, le mélange de ligation est tout d'abord extrait avec un mélange phénol/chloroforme puis précipité avec de l'éthanol avant coupure spécifique avec les enzymes de restriction appropriées puis précipitation avec le tétrachlorhydrate de spermine.

Les cellules bactériennes compétentes sont préparées puis transformées avec les plasmides ou transfectées par l'ADN de M13 selon les procédés décrits par Dagert et Ehrlich (1979).

Les plasmides de levures sont introduits dans les levures suivant la technique aux sels de lithium de Ito et coll. 1983.

La sélection est faite sur un milieu minimum synthétique plus histidine (40 ug/ml) pour les segregeants mentionnés précédemment.

EXEMPLE 1

<u>Préparation de pTG320, vecteur d'expression dans les bactéries</u>

La synthèse du plasmide pTG320 nécessite d'une part la préparation d'un plasmide vecteur, pTG951.

Ce plasmide pTG951 a été obtenu par une stratégie complexe.

La synthèse de ce vecteur, qui est développée dans le brevet français n° 83 19777 déposé le 9 décembre 1983 par la demanderesse, ne sera que brièvement rappelée en regard des schémas ci-annexés.

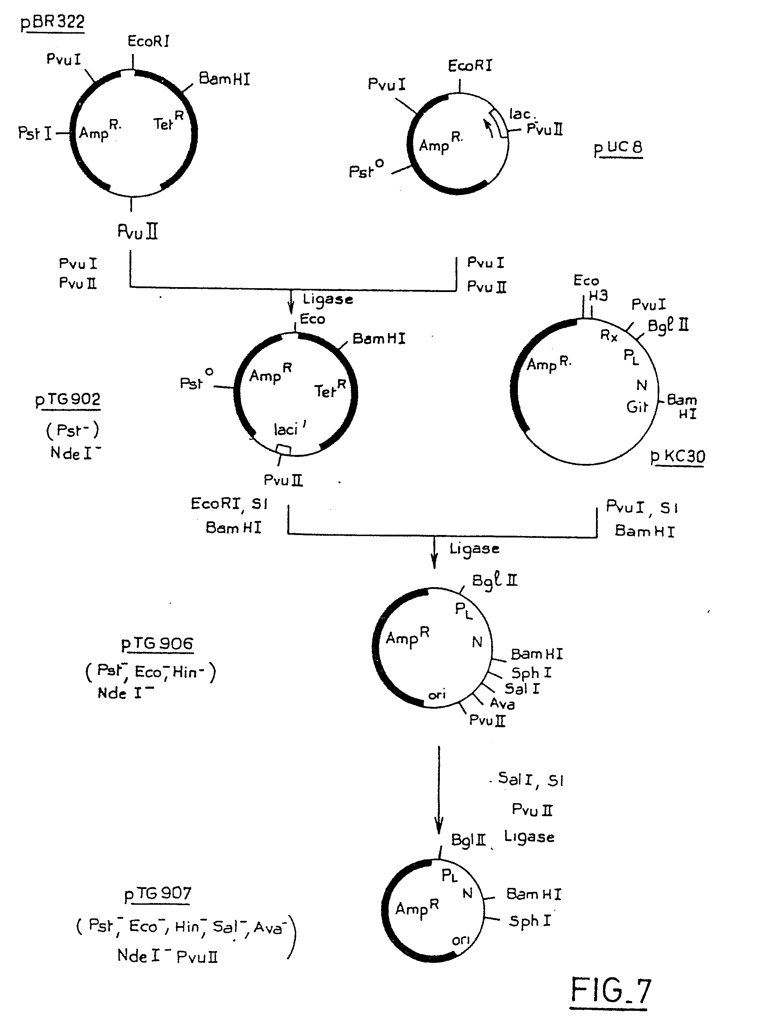

a) <u>Construction de pTG908</u> (fig. 7, 8 et 9)

Le plasmide de base utilisé est le plasmide pBR322 ; toutefois, celui-ci présente l'inconvénient d'avoir à l'intérieur du gène Amp$^r$ un site de restriction PstI, car un site de même nature sera utilisé par la suite dans la zone de clonage comme site unique de restriction. Il convient donc de faire disparaître ce site de restriction PstI en utilisant un mutant du plasmide pBR322, le plasmide pUC8, dans lequel le gène de résistance à l'ampicilline ne présente pas de site de restriction PstI (ce site a été éliminé par mutation in vitro). pBR322 est commercialisé notamment par Bethesda Research Laboratories et pUC8 est décrit dans l'article de Vieira J. et Messing J.

Pour ce faire, on échange le fragment PvuI/PvuII de 1 669 bp de pBR322 avec le fragment analogue PvuI/PvuII du plasmide pUC8. Afin de réaliser cet échange les plasmides pBR322 et pUC8 sont traités successivement par PvuI, PvuII, puis circularisés par action d'une ligase.

On obtient ainsi le plasmide pTG902 qui ne présente plus de site de restriction PstI et qui a également perdu le site de restriction NdeI présent à l'origine sur pBR322 (non représenté sur la figure 7). En outre, le plasmide pTG902 porte un fragment de 50 bp correspondant à la séquence laci' dans lequel se trouve le site PvuII.

Le promoteur $P_L$ et le gène $\lambda$N (qui provient du phage $\lambda$, le gène $\lambda$N code pour une fonction d'antiterminaison de transcription) sont isolés du plasmide pKC30 pour être insérés dans pTG902 comme cela est indiqué sur la figure 7 ci-annexée par traitement EcoRI, S1, BamHI pour pTG902 et par traitement PvuI, S1, BamHI pour pKC30 avec ligation.

L'un des plasmides obtenus après transformation de la souche TGE900, pTG906, est traité de façon à éliminer le segment PvuII-SalI. Pour ce faire, pTG906 est traité successivement avec SalI, la nucléase S1, PvuII et la ligase. On obtient ainsi pTG907.

On insère ensuite la "région de fixation des ribosomes" $\lambda$cIIrbs (provenant elle aussi du phage $\lambda$) sous forme d'un fragment AvaI/TaqI dans le début du gène lacZ' (fragment a de la $\beta$-galactosidase) lequel a été cloné dans le phage M13 nommé M13tg110. Cette stratégie permet un test fonctionnel simple pour rbs, à savoir la production de la protéine lacZ' et, par conséquent, d'obtenir des plaques bleues en présence d'IPTG et de Xgal ; ceci permet également un séquençage rapide de la construction en utilisant la méthode dite du didéoxy.

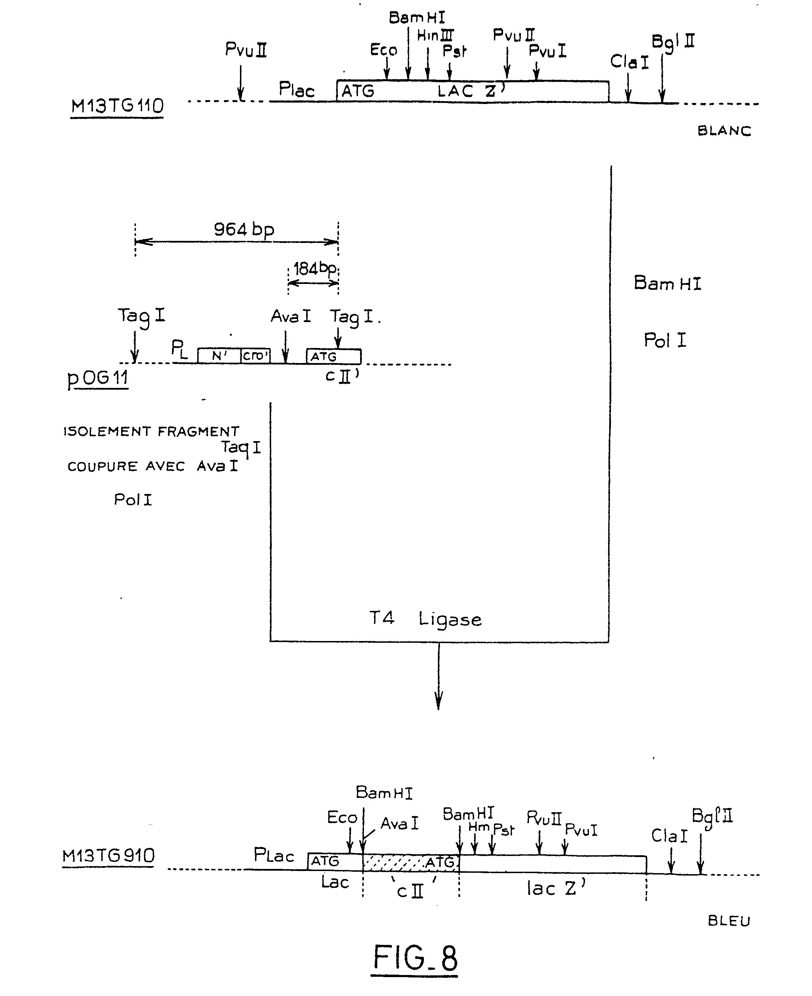

On obtient ainsi après sélection dans les bactéries compétentes un clone résultant M13tg910 dont la structure globale est représentée à la partie inférieure de la figure 8.

Le fragment cIIrbs/lacZ' du phage M13tg910 est transféré sur le plasmide vecteur pTG907 préparé précédemment.

Pour ce faire, on élimine les sites EcoRI, BamHI et AvaI en amont de cIIrbs puis on insère un site BglII.

Dans ces conditions, cIIrbs peut être prélevé sous forme d'un fragment BglII-BglII et placé dans le site BamHI en aval du promoteur $P_L$ et du gène N de pTG907.

Le phage M13tg910 est digéré avec EcoRI puis traité avec Bal31 puis ensuite par la polymérase de Klenow. Les fragments obtenus sont alors soumis à l'action de la ligase en présence d'adaptateurs BglII non phosphorylés. Le mélange de ligation obtenu est utilisé pour transformer des cellules compétentes JM103.

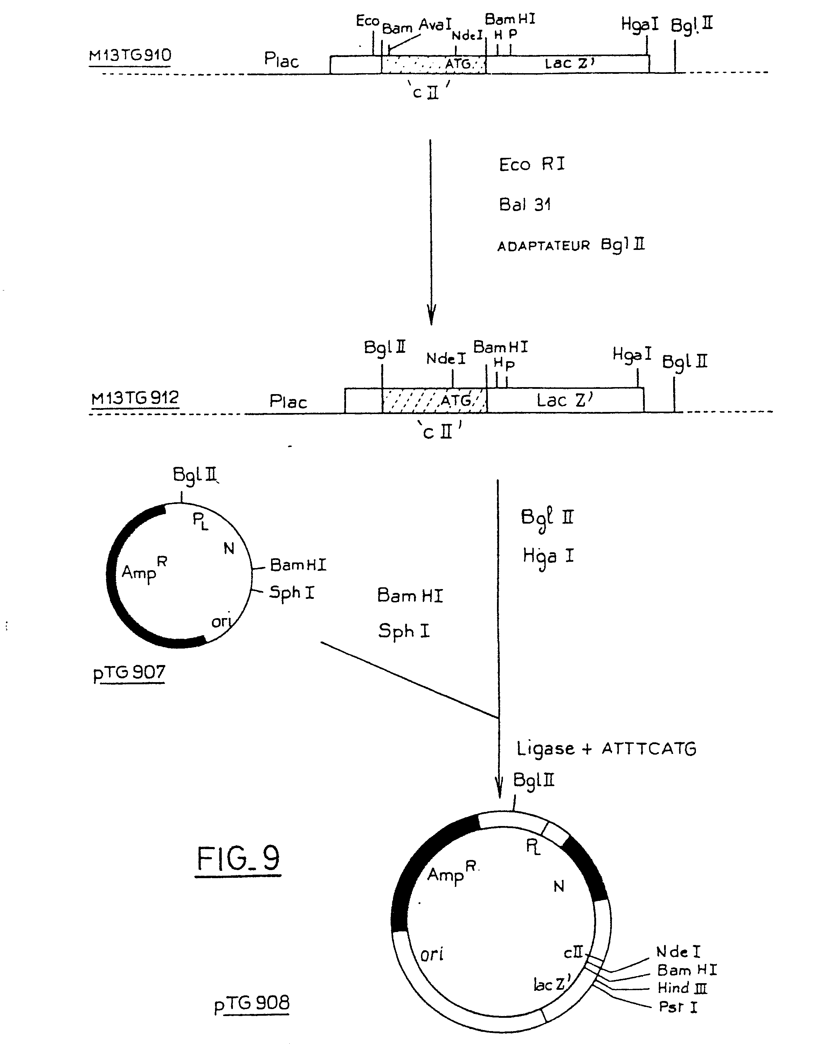

On sélectionne alors les plages bleues. Ces clones sont ensuite analysés afin de vérifier qu'ils contiennent le site BglII et qu'ils ne présentent plus de site EcoRI ou BamHI en amont. On obtient ainsi des clones tels que M13tg 912 dont la structure est représentée sur la figure 9 .

Le traitement par Bal31 a produit une délétion de 101 bp éliminant les sites EcoRI, BamHI et AvaI ; ainsi que les séquences de lac ATG et lac Shine/Dalgarno. Le site BglII introduit se trouve placé enrion 100 bp en amont de l'ATG de cII et 10 bp en aval de $P_{lac}$.

Le fragment BamHI/SphI de pTG907, le fragment BglII/EpaI portant cIIrbs et lacZ' et l'adaptateur phosphorylé ont été préhybridés dans un rapport molaire de 1:2:1 puis traités avec la ligase $T_4$. Des aliquots sont utilisés pour transformer les cellules compétentes de la souche 6150 à 30°C.

Les cellules intéressantes sont identifiées en sélectionnant les transformants avec un fragment cIIrbs/lacZ' marqué au $P^{32}$ et la construction obtenue est confirmée par une étude de restriction enzymatique.

Afin d'avoir une première indication montrant que les différents éléments du système d'expression se conduisent comme cela est désiré, le plasmide obtenu, pTG908, est transféré dans une souche hôte N6437 qui possède à la fois cl857 et le fragment ω de la β-galactosidase complémentant le fragment α qui est codé par le plasmide.

Les transformants obtenus placés sur une boîte contenant IPTG + Xgal sont blancs à 28°C puis virent au bleu environ 30 minutes après lorsqu'on les transfère à 42°C.

Ce vecteur avant d'être utilisé pour cloner FIX a été adapté pour cloner l'interféron γ humain : IFN-γ, en fait pour le clonage de FIX la nature de la protéine intermédiaire clonée n'a pas d'importance, mais les vecteurs ont été réalisés selon le schéma ci-après.

L'analyse de la séquence des nucléotides de IFN-γ pour les sites de restriction révèle un site EcoRII à 8 bp en aval du départ de la protéine mature et un site Sau3A à 285 bp en aval du codon stop, ce qui permet d'isoler pratiquement toute la séquence codant pour la protéine mature sur un fragment EcoRII/Sau3A. Le clone de IFN-γ obtenu à partir d'une banque est nommé pTG11.

b) Construction de pTG909

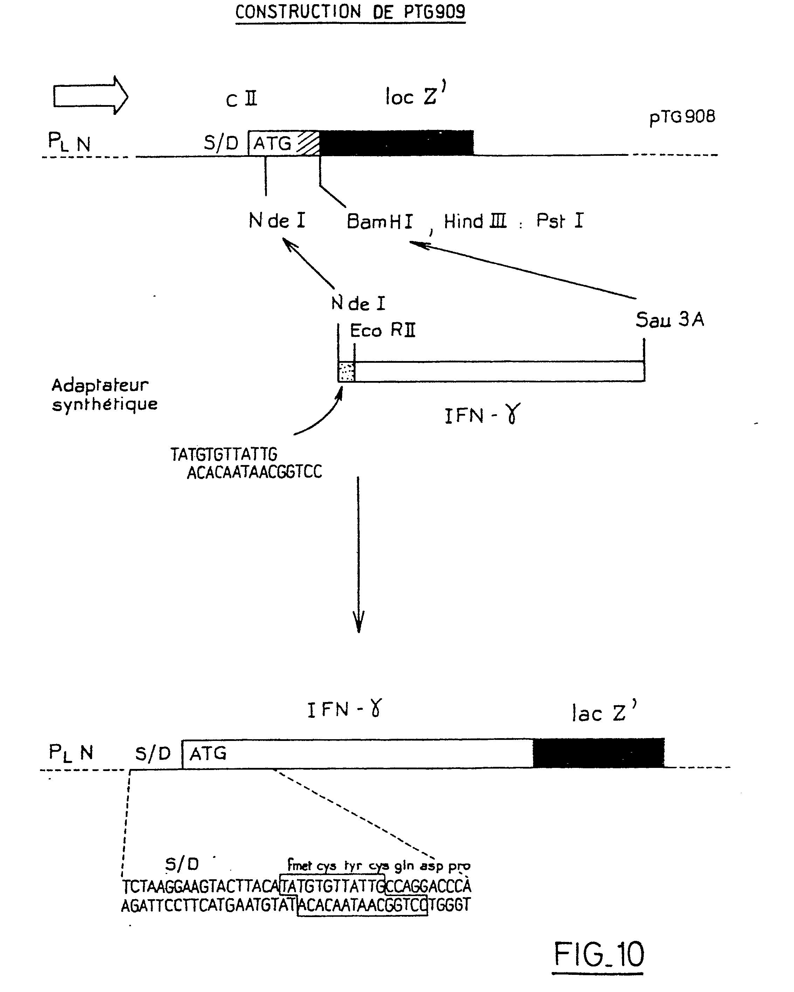

La figure 10 schématise la préparation de pTG909.

On utilise tout d'abord une molécule d'adaptation synthétique qui permet :

a) d'effectuer la jonction entre les extrémités EcoRII et NdeI,

b) d'introduire les 8 bp manquantes par rapport à la séquence codant pour IFN-γ mature et,

c) de reconstituer le codon de départ ATG de cIIrbs de façon que la séquence codant pour la protéine IFN-γ mature soit traduite sans amino-acides fusionnés à l'exception de l'initiateur F-met.

Cet adaptateur est synthétisé chimiquement et sa constitution est représentée sur la figure 10.

pTG11 est mis en digestion avec EcoRII et Sau3A et pTG908 avec NdeI et BamHI.

Les fragments appropriés sont purifiés sur gel, mélangés avec une quantité équimolaire de l'adaptateur, préhybridés et ligés. Le mélange est utilisé pour transformer des cellules compétentes TGE900 et les transformants sont sélectionnés en hybridant un insert PstI de pTG11 "nick-traduit" et marqué au p$^{32}$ avec les transformants.

13 clones sont sélectionnés et contrôlés par cartographie et l'un d'eux pTG909 est vérifié par séquençage.

### c) Construction du vecteur pTG941

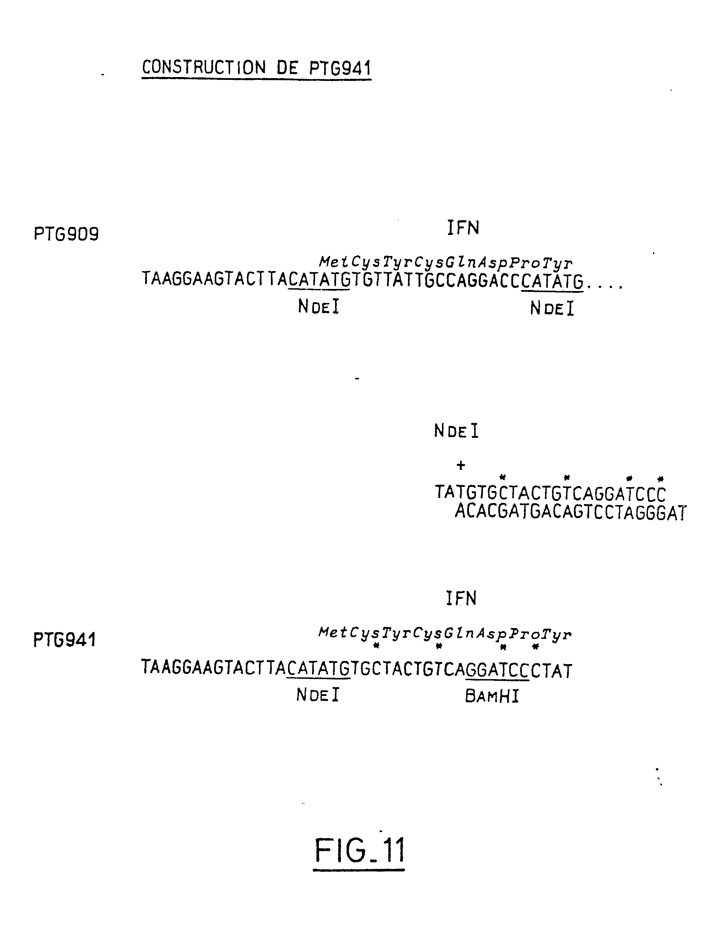

pTG909 contient 2 sites NdeI, l'un au codon de départ de IFN-γ et l'autre 22 bp en aval de la séquence IFN-γ (voir figure11).

La région entre ces sites qui est la région codant pour les 7 premiers amino-acides de IFN-γ a été éliminée par traitement avec NdeI et remplacée par un oligonucléotide synthétique qui est représenté sur la figure 11.

Cette réaction détruit le site NdeI aval et reconsti-tue le site NdeI amont tout en introduisant un site BamHI qui est unique. On obtient ainsi le vecteur pTG941.

### d) Construction de pTG951

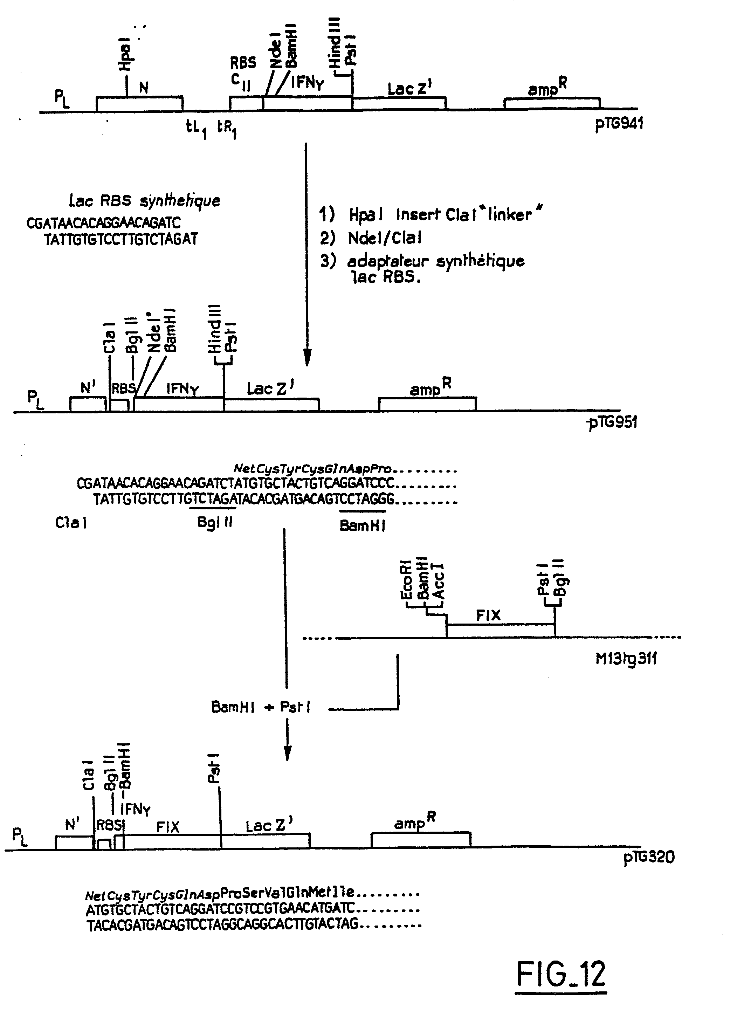

La figure 12 schématise la construction de pTG951 qui est un dérivé de pTG941 dans lequel le fragment contenant le cIIrbs a été remplacé par une séquence synthétique sur la base de la séquence de la région d'initiation de la traduction de l'opéron E. coli lac noté E. coli lac opéron rbs. Cet oligonucléotide synthétique a été inséré au niveau du site HgaIentre le site unique NdeI du codon de départ de la séquence codant pour IFN-γ et le site ClaI qui a été inséré dans le gène N.

De ce fait par traitement avec <u>NdeI</u> et <u>ClaI</u> le plasmide pTG951 ne contient plus qu'un gène <u>N</u> tronqué (un codon stop en phase avec la traduction du gène <u>N</u> est placé immédiatement en amont du nouveau site rbs) et est dépourvu des terminateurs de transcription tL1 et tR1 présents dans pTG909 et pTG941.

Le site rbs synthétique de pTG951 contient un site <u>Bgl</u>II immédiatement avant le codon de départ, il est donc possible de faire des dérivés de pTG951 par scission avec <u>Bgl</u>II suivi par diverses manipulations utilisant soit l'ADN polymérase I ou la nucléase S1. Ces dérivés présentent des variations dans la distance et les séquences entre la séquence de Shine/Dalgarno et l'ATG. Mais aucun des plasmides ainsi préparé ne présente une activité supérieure au pTG951 lui-même.

Les principaux résultats sont rappelés dans le tableau ci-après :

| NOM | PROMOTEUR | RBS | SEQUENCE DE RBS ET JONCTION AVEC LA SEQUENCE |
|---|---|---|---|
| pTG909 | PL | cII | fmet cys tyr cys gln asp pro<br>TAAGGAAGTACTTACATATG TGT TAC TGC CAG GAC CCA |
| pTG941 | PL | cII | fmet cys tyr cys gln asp pro<br>TAAGGAAGTACTTACATATG TGC TAC TGT CAG GAT CCC |
| pTG951 | PL | SYNTH (lac) | fmet cys tyr cys gln asp pro<br>CACAGGAACAGAGATCTATG TGC TAC TGT CAG GAT CCC<br>------<br>BglII |

La construction de pTG320 est schématisée à la figure 12.

pTG951 comporte un site de clonage avec de nombreux sites uniques de restriction situé en aval du promoteur et du site de fixation des ribosomes. Immédiatement en aval du site de fixation des ribosomes lac synthétique se trouve situé un site BglII. Lorsqu'un gène hétérologue est cloné dans ce site, les sites de restriction uniques additionnels peuvent être introduits. Ces sites peuvent alors être employés pour exprimer des gènes hétérologues additionnels. Comme pTG951 contient une séquence d'interféron humain qui contient un site unique BamHI, 21 paires de base en aval du site BglII un gène hétérologue peut être aussi cloné dans ce site. Le choix du site BglI1 ou du site BamHI à utiliser dépend du gène hétérologue qui doit posséder un ATG initiateur immédiatement en aval des extrémités cohésives de BglII BamHI. Si comme cela est le cas dans le facteur IX ceci n'est pas possible, le site BamHI doit être utilisé et la protéine sera exprimée sous forme fusionnée avec 5 des aminoacides de l'interféron humain.

Pour l'expression du facteur IX dans E. coli pTG951 a été mis en digestion avec BamHI et PstI, éliminant ainsi les 5 aminoacides de la séquence de l'interféron γ et insertion du fragment BamHI/PstI provenant de M13tg311. La séquence du plasmide résultant, pTG320, au niveau de la fusion interféron γ facteur IX est la suivante :

```
                     M   C   Y   C   Q   D   P   Y                                    A   P   S   V   Q   M   I   M   A
pTG951      A^GATCTATGTGCTACTGTCAG^GATCCCTAT        M13tg311        G^GATCCGTCCGTGAACATGATCATGGCA........
            /                      /                                /              -------------------------------------
           /                      /              +                 /
          BglII                  BamHI                            BamHI


                               1           5                10
                               M   C   Y   C   Q   D   P   S   V   Q   M   I   M   A
        pTG320      A^GATCTATGTGCTACTGTCAG^GATCCGTCCGTGAACATGATCATGGCA................
                    /                      /                 --------------------------------------
                   /                      /
                  BglII                  BamHI
```

Les lettres au-dessus des séquences de nucléotide se réfèrent au code conventionnel
à une lettre pour les amino-acides codés.

La séquence de facteur IX est soulignée.

Comme cela est indiqué, la traduction du mARN de facteur IX commence à l'ATG venant
de l'extrémité 5' de l'interféron γ . La protéine résultante est ainsi une protéine
fusionnée avec 8 amino-acides fusionnés à la séquence signal incomplète du facteur IX.

EXEMPLE 2

Expression du facteur IX dans la levure

Le vecteur d'expression utilisé est le plasmide
pTG832.

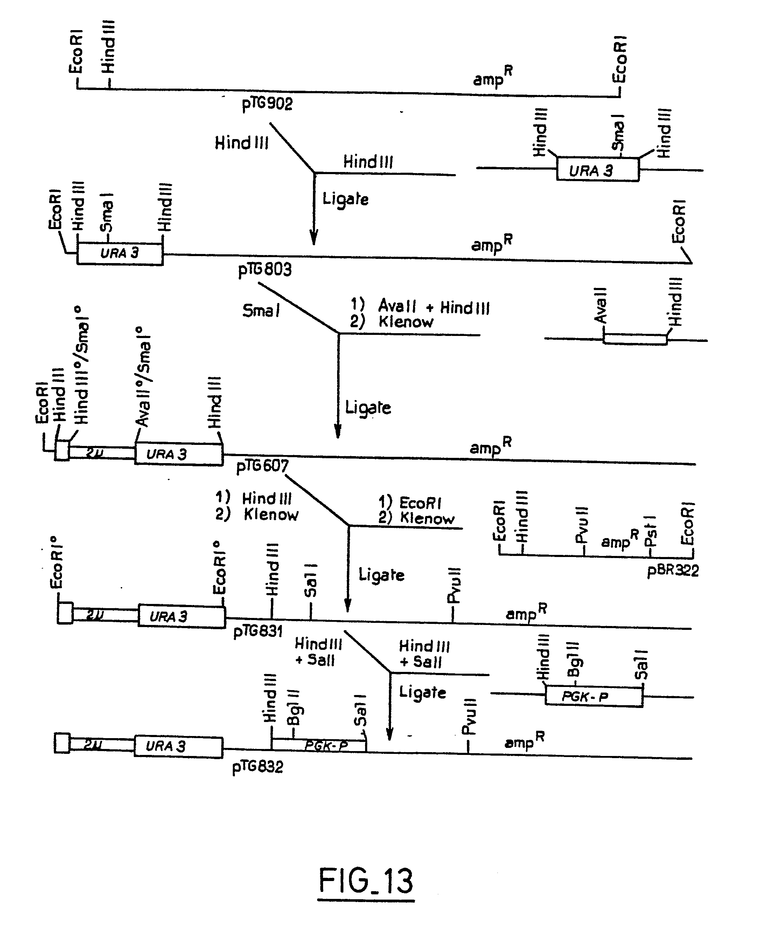

La construction du plasmide pTG832 est rappelée
dans la figure 13.

Le plasmide de départ est le plasmide pTG902
obtenu à partir de pBR322 par délétion des sites PstI
et NdeI.

Par digestion de pTG902 et du plasmide portant le gène ura3 (ne possédant pas de site PstI) avec HindIII et ligation du produit de digestion, on obtient le plasmide pTG803.

Le plasmide 2μ de levure est mis en digestion avec AvaII et HindIII et les extrémités sont rendues franches par traitement avec la polymérase de Klenow. Ce fragment de 2μ AvaII/HindIII est ligué avec pTG803 digéré par SmaI pour fournir le plasmide pTG807.

Le gène ura3 et l'origine de réplication du 2μ sont libérés sous forme d'un fragment unique par digestion de pTG807 avec HindIII.

Ce fragment est traité par la polymérase de Klenow et cloné dans le site HindIII de pBR322 traité de façon similaire pour donner pTG831.

Le gène de levure phosphoglycerate kinase (PGK) est muté de façon que le site unique BglII soit introduit à l'ATG d'initiation de la traduction de la protéine PGK.

Le fragment HindIII/SalI (2,15 kb), comprenant le promoteur et le début de la région codante du gène correspondant à la phosphoglycérate kinase de levure, a été cloné dans les mêmes sites du vecteur M13mp8.

La création d'un site BglII au niveau de l'ATG initiateur de la phosphoglyceratekinase a été effectuée suivant les techniques classiques de mutagénèse in-vitro (Gillam and Smith, 1979 gene 8, 99-106).

Pour ce faire, un oligonucléotide synthétique, de séquence 5' ATATAAAACAAGATCTTTATC 3', a été utilisé afin de modifier la séquence originale 5' ATATAAAACAATGTCTTTATC 3' ; l'ATG initiateur est ainsi rendu non fonctionnel du fait de son remplacement par la séquence AGA.

Ce gène ainsi modifié est cloné dans le plasmide pTG831 pour donner le plasmide pTG832.

Le terminateur de PGK situé dans le fragment
EcoRI/HindIII est cloné dans le site PvuII de pTG832
après traitement-de ses extrémités à la polymérase de
Klenow. Le plasmide résultant, pTG833, est mis en
digestion avec BglII et recircularisé pour donner le
vecteur d'expression final pTG834. Comme cela est indiqué
précédemment, le site unique BglII dans ce vecteur est
utilisable pour l'expression de gène hétérologue dans la
levure. C'est dans ce site que le fragment de cADN
BamHI/BglII provenant de M13tg311 et les fragments de
cADN FIX BamHI de M13tg315 ont été clonés pour donner
respectivement pTG318 et pTG324.

EXEMPLE 3

Expression du facteur IX dans les cellules de mammifère.

1) le plasmide pMOP

Le virus BPV se divise en deux parties limitées
par deux sites uniques de restriction HindIII et BamHI
qui délimitent deux fragments dits respectivement de
"69 %" et de "31 %".

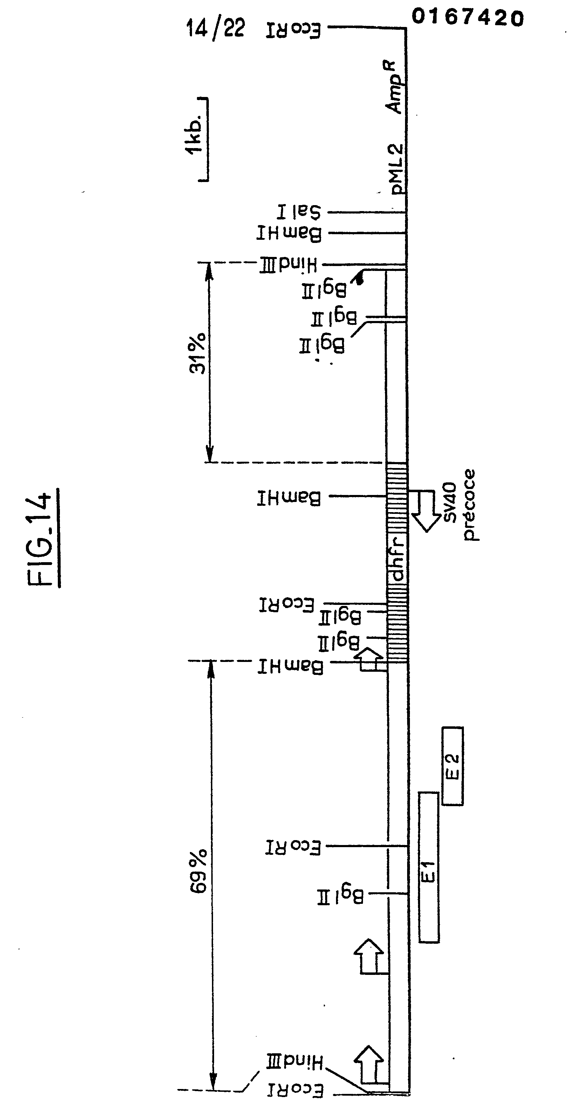

Le vecteur dérivé du BPV mis en oeuvre dans
cet exemple est dénommé pMOP. Il est obtenu par introduction dans le site BamHI du BPV du marqueur dhfr codant
pour la dihydrofolate réductase sous le contrôle du
promoteur précode de SV40 (voir figure 14).

Ce vecteur pMOP comporte en outre un fragment
de pBR322 portant le gène de résistance à l'ampicilline
qui provient du plasmide pML2 décrit par Lusky et Botchan,
1981.

Ce plasmide vecteur a été préparé à l'origine
afin de cloner la séquence codant pour une protéine
antigénique de la rage. comme cela est décrit dans le
brevet français n° 83 15716.

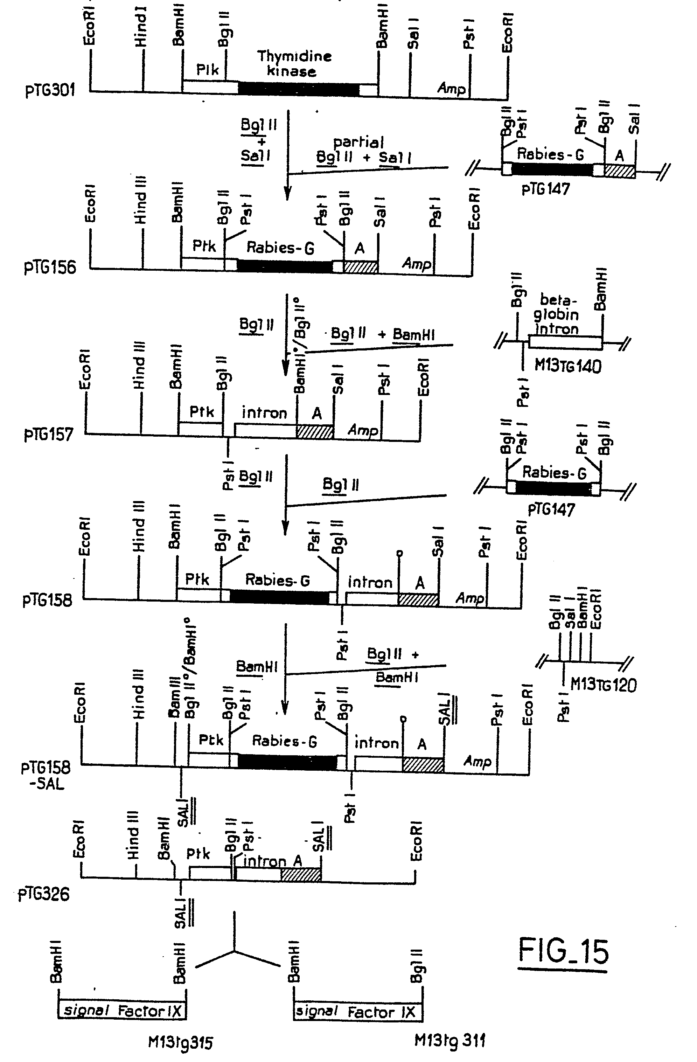

2) Le plasmide pTG158SAL (figure 15)

Le gène codant pour la protéine antigénique de la rage provient du plasmide pTG147 dont la construction a été explicitée dans le brevet français mentionné précédemment.

Ce gène est prélevé sous forme d'un fragment BglII/SalI par digestion partielle de pTG147 pour être introduit entre les sites correspondants du plasmide pTG301 traité par BglII/SalI. Ce plasmide pTG301 comporte le gène de la thymidine kinase (tk) et son promoteur (ptk), il est obtenu par insertion dans le site BamHI de pBR322 d'un fragment BamHI-BamHI de l'Herpes virus simplex portant tk et ptk, le traitement de restriction mentionné élimine le gène tk.

Le produit de ligation pTG156 comporte dans sa partie intéressante le gène de la rage sous le contrôle du promoteur de la thymidine kinase, le gène étant suivi du site de polyadénylation de SV40 (A).

L'introduction de l'intron de la globine est effectuée à partir du phage M13tg140, dont la construction est décrite dans le brevet français, sous forme d'un fragment BglII/BamHI qui est inséré dans le site BglII de pTG156 après délétion du fragment BglII/BglII correspondant. Cette délétion ayant supprimé le gène de la rage, celui-ci est réintroduit dans le site unique BglII de pTG156 sous forme d'un fragment BglII/BglII de pTG147. On obtient ainsi le plasmide pTG158.

La partie essentielle de ce plasmide devant être prélevée sous forme d'un fragment SalI/SalI (l'un des rares sites uniques de pMOP) on introduit en amont du promoteur au niveau du site BamHI un petit fragment provenant de M13tg120 et comportant un site SalI. M13tg120 est mis en digestion avec BglII et BamHI et pTG158 avec BamHI, l'ensemble est lié pour donner pTG158 SAL.

3) <u>Synthèse du vecteur</u> (figure 15)

Par digestion de pTG158 et de pMOP avec SalI et ligation on obtient le plasmide pTG172 qui comporte, outre l'ensemble des éléments rappelés pour pMOP, le gène de la rage sous le contrôle du promoteur Ptk et terminé par l'intron de la β globine et un site de polyadénylation.

Pour l'expression du facteur IX pTG158SAL est mis en digestion avec BglII et recircularisé pour donner pTG326. Ce plasmide contient tous les éléments essentiels pour l'expression d'un gène hétérologue dans une cellule de mammifère :

. promoteur intron et site de polyadénylation.

Un site unique BglII est placé entre le promoteur et l'intron, ce qui permet l'expression de gènes que l'on y clone. C'est dans ce site que le fragment de cADN BamHI/BglII de FIX provenant de M13tg311 et le fragment BamHI de FIX de M13tg315 ont été clonés pour donner respectivement pTG317 et pTG325.

4) <u>Expression du facteur IX</u>

a) <u>Expression dans les bactéries</u>

Comme cela a été indiqué précédemment, la transcription à partir du promoteur de PL de λ est contrôlée par une protéine cI857 thermolabile codée par la cellule hôte. A 30°C la transcription à partir du promoteur PL est inhibée compte tenu de l'effet de régulation négatif de la protéine cI857. A une température supérieure (supérieure à 35°C) la protéine cI857 thermolabile se dénature, elle libère ainsi le contrôle négatif de la transcription à partir de PL. Les produits protéiniques de la fermentation de la souche de <u>E. coli</u> TG900 transformée par divers vecteurs est effectué  par marquage et immunoprécipitation.

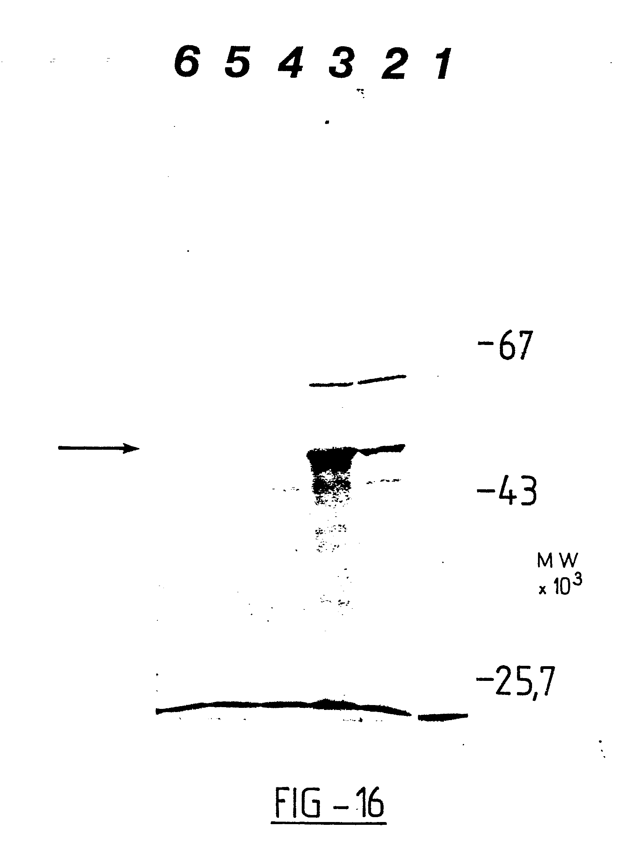

La figure 16 montre les produits protéiniques marqués à la méthionine $S_{35}$ 20 $\mu$Ci/ml pendant 1 minute et précipités avec un anti-sérum de lapin anti-facteur IX.

La ligne 1 correspond au standard de poids moléculaire.

Les lignes 2, 3 et 4 correspondent au plasmide pTG320 (facteur IX).

Les lignes 5 et 6 correspondent au vecteur plasmidique pTG951.

Les lignes 4 et 6 correspondent à des cultures à 30°C.

Les lignes 2, 3 et 5 correspondent à des cultures à 37°C.

La ligne 2 est la même que la ligne 3, sauf que l'immunoprécipitation est effectuée en présence de 10 $\mu$ g de facteur IX comme compétiteur.

Comme cela est indiqué par la flèche, l'induction de pTG320 à 37°C produit une protéine qui est spécifiquement immunoprécipitée par l'antisérum de lapin anti-facteur IX. Le poids moléculaire apparent de cette protéine est de 47 000 daltons. L'antisérum utilisé a été préparé en utilisant une préparation de facteur IX natif purifié.

Ceci a été confirmé puisque la protéine du facteur IX purifié non marquée peut entrer en compétition au niveau de l'immunoprécipitation avec la protéine de 47 000 daltons (ligne 1). Il faut mentionner que le facteur IX produit par les bactéries à la différence du facteur IX natif utilisé pour préparer l'antisérum est ni glycosylé ni $\gamma$-carboxylé. En dépit de ceci, le facteur IX produit par les bactéries est reconnu par l'antisérum de lapin anti-facteur IX natif.

La taille espérée pour le facteur IX produit dans cette construction est de 51 300 daltons. Ceci tient compte des 8 amino-acides de l'interféron ɣ qui sont fusionnés à la séquence signal du facteur IX.

La taille observée pour le facteur IX bactérien est significativement plus petite que la taille espérée et correspond de façon plus proche à la protéine mature dans lequel on a supprimé la séquence signal et la préprotéine (46 000 daltons). Ainsi, le mécanisme de séparation spécifique qui est responsable de l'élimination de la séquence signal et de la séquence de la préprotéine du facteur IX pourrait être fonctionnelle dans E. coli.

### Expression de facteur IX dans Saccharomyces cerevisiae

Les expériences suivantes sont effectuées pour déterminer si les cellules comportant le plasmide recombinant pTG318 synthétise le facteur IX.

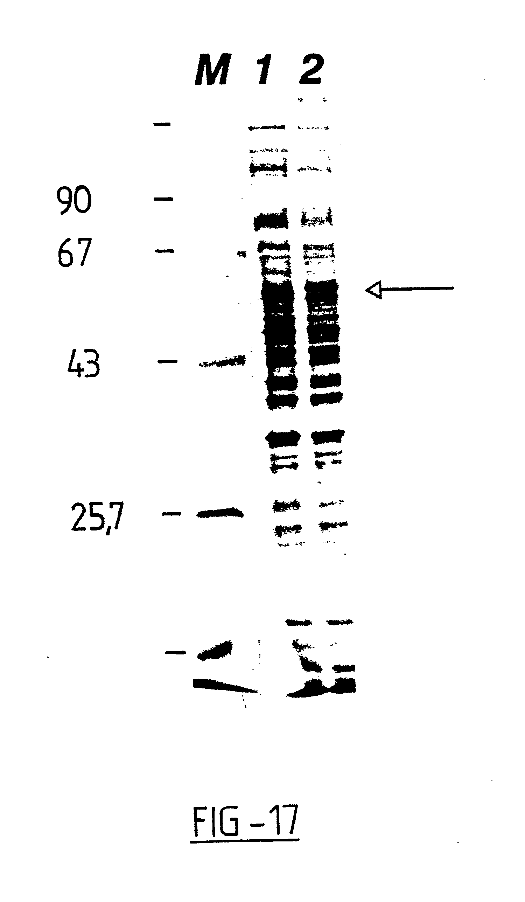

La culture en phase logarithmique de pTG834 (vecteur) et pTG318 (facteur IX) est effectuée en milieu minimum jusqu'au milieu de la phase logarithmique. Un aliquote de 5 ml est prélevé pour chaque culture et les protéines sont marquées avec de la méthionine S35 pendant 20 minutes à 30°C. Les cellules sont récoltées par centrifugation, remises en suspension dans 0,5 ml TGE qui contient un mélange inhibiteur de protéase puis lysées par la méthode des billes de verre. Après clarification par centrifugation, les protéines dans les extraits cellulaires sont analysées par électrophorèse sur gel de polyacrylamide SDS. Le profil des protéines synthétisées est indiqué à la figure 17:

. ligne 1 pTG834

. ligne 2 pTG318

. M standard de poids moléculaire.

0167420

La flèche indique la position de la bande (poids moléculaire 57 000 daltons) qui est particulièrement intense en particulier dans le facteur IX obtenu à partir de pTG318.

La taille de cette protéine est en accord avec la taille espérée, si la forme mature du facteur IX plus le signal (50 600 daltons) est glycosylée, un procédé qui apparaît dans les levures et qui est connu pour ajouter à peu près 7 000 daltons au poids moléculaire apparent du facteur IX. On trouve une répartition à peu près similaire des protéines pour le plasmide pTG324 qui contient, en outre, 5 amino-acides additionnels à l'extrémité N terminal du peptide signal de facteur IX.

D'autres informations confirment l'expression du facteur IX dans les levures portant les plasmides recombinants pTG318 et pTG324 et proviennent d'une analyse ELISA des extraits cellulaires. A titre de contrôle, les extraits cellulaires portant le plasmide pTG834 ont également été testés et il a été démontré qu'il était incapable de préparer l'antigène du facteur IX. Ces expériences ont été effectuées à l'aveugle, l'expérimentateur ne connaissant pas les sources et les préparations des échantillons qui étaient analysés par ELISA. Les résultats de cette analyse sont représentés au tableau I. La méthode employée pour la détermination quantitative de l'antigène de facteur IX est décrite.

## L'expression du facteur IX dans les cellules de mammifères

Des lignées cellulaires de cellules de mammifères sont transformées avec le plasmide pMOP ou les dérivés pTG317 ou pTG325 contenant le facteur IX dans le bloc d'expression fourni par le plasmide pTG326. 4 lignées cellulaires ont été transformées :

NIH-3T3 : (fibroblaste d'embryon de souris) ;

LMTK    : (fibroblaste d'embryon de souris) ;

MDBK    : (rein de bovins)

VERO    : (rein de singe).

Des résultats ont été obtenus pour les cellules NIH-3T3 et LMTK et sont actuellement en attente pour les lignées MDBK et VERO.

Comme cela a été indiqué précédemment, le vecteur employé pour exprimer le facteur IX dans les cellules de mammifères (pMOP) permet la sélection de transformants stables en présente de l'antimétabolite méthotrexate. Ainsi après transformation des cellules par la technique standard utilisant la précipitation de l'ADN au phosphate de calcium, les transformants sont sélectionnés en présence de 0,4 µg/ml de méthotrexate. Les colonies de cellules transformées sont détectées visuellement sans l'aide de microscope au bout de deux à trois semaines. Ces colonies peuvent alors être isolées, cultivées et analysées séparément.

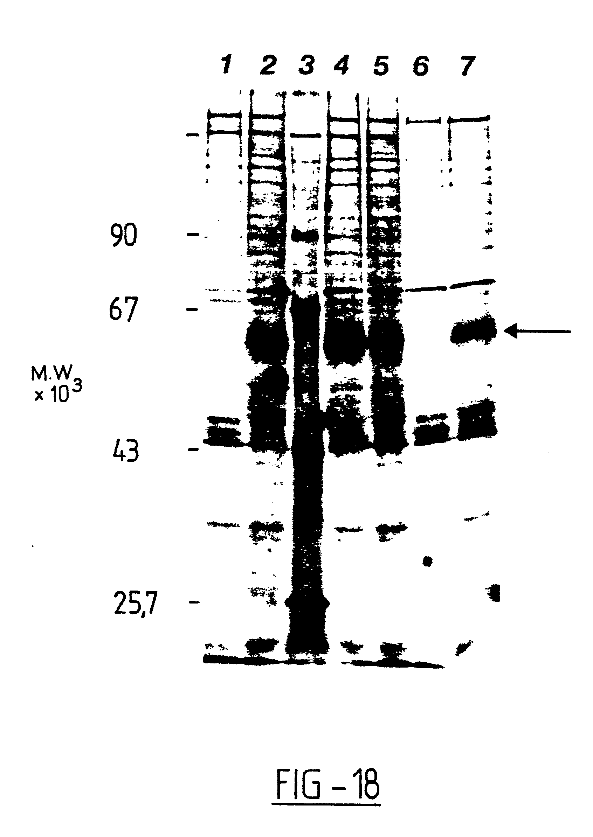

Le résultat d'une immunoprécipitation des protéines marquées à la méthionine S35 avec un sérum de lapin anti-facteur IX natif pour différentes colonies isolées de cellules de NIH-3T3 portant le plasmide pTG317 est représenté à la figure 18:

ligne 1 contrôle non transformé cellule 3T3,

ligne 2 mélange de différentes colonies,

ligne 3 marqueur de poids moléculaire,

ligne 4 pTG317 clone 7,

ligne 5 pTG317 clone 1,

ligne 6 pTG317 clone 9,

ligne 7 pTG317 clone 4,

Comme cela est indiqué dans les lignes 2, 4, 5 et 7, on observe une expression très forte d'une protéine de 62 000 daltons immunoprécipitée spécifiquement avec un anti-sérum de lapin anti-facteur IX. La présence ou l'absence de cette protéine est en correlation avec la détection de l'antigène du facteur IX dans les extraits cellulaires analysés par ELISA.

La protéine de 62 000 daltons ne forme pas une bande aiguë dans les conditions d'électrophorèse utilisées. Comme cette glycoprotéine forme une bande diffuse caractéristique sur gel de polyacrylamide SDS, ce résultat suggère que le facteur IX obtenu à partir des cellules 3T3 transformées par pTG317 peuvent être glycosylées.

De façon à déterminer si la protéine de 62 000 daltons est une glycoprotéine, les extraits cellulaires marqués à la méthionine S35 de 3T3.pTG317.clone 7 (ci-après 3T3.317.7) sont mis en incubation avec la concanavaline A-Sépharose (Pharmacia). Dans les conditions employées, la glycoprotéine est spécifiquement absorbée au dérivé de Sépharose. Le facteur IX restant dans le surnageant après incubation avec ConA-Sépharose est déterminé par immunoprécipitation et est comparé à la quantité de facteur IX immunoprécipitable avant incubation avec ConA-Sépharose.

Dans ces conditions, la protéine de 62 000 daltons est quantitativement éliminée de l'extrait 3T3.317.7 par absorption sur ConA-Sépharose.

Le fait que cette absorption ne soit pas due à une absorption non spécifique des protéines sur ConA-Sépharose est démontrée car seule le protéine de 62 000 daltons est prélevée alors que les protéines de l'arrière plan demeurent dans le surnageant.

L'analyse de protéine marquée à la méthionine S35 sans immunoprécipitation montre la présence d'une protéine de 72 000 daltons dans tous les cas dans laquelle un antigène de facteur IX est détecté par ELISA et immunoprécipitation d'une protéine de 62 000 daltons.          L'identité de cette protéine de 72 000 daltons est incertaine ; toutefois elle est clairement associée avec la présence de l'antigène de facteur IX dans les cellules 3T3 transformées. Il est possible que cette protéine représente un précurseur biosynthétique de la protéine de 62 000 daltons. Les expériences qui vont être décrites démontrent que cette protéine de 72 000 daltons est spécifiquement immunoprécipitée par un anti-sérum de lapin anti-facteur IX.

La résistance au méthotrexate dans 3T3.317.7 provient de l'expression du gène de la dihydrofolate réductase (dhfr) portée par le plasmide. Si la concentration de méthotrexate est accrue, une certaine proportion des cellules répondra en augmentant le nombre de copies du gène de dhfr. Comme les séquences de dhfr et du facteur IX sont situées sur le plasmide pTG317, on pense pouvoir ainsi, en augmentant la dose de méthotrexate, co-amplifier l'expression de dhfr et du gène de facteur IX. Pour tester cette hypothèse , le méthotrexate est ajouté à la culture de 3T3.317.7 à une concentration variant entre 0,4 et 8 µg/ml.

Après plusieurs semaines de culture à ces concentrations, les cellules sont marquées avec de la méthionine S35 comme cela est décrit précédemment et les protéines analysées sur gel de polyacrylamide SFS.

**0167420**

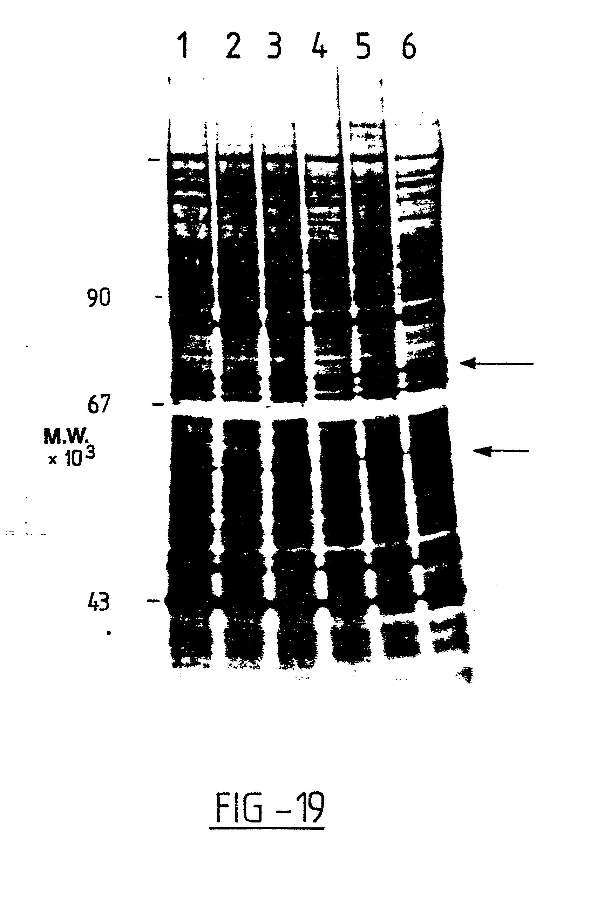

La figure 19 montre les résultats obtenus.

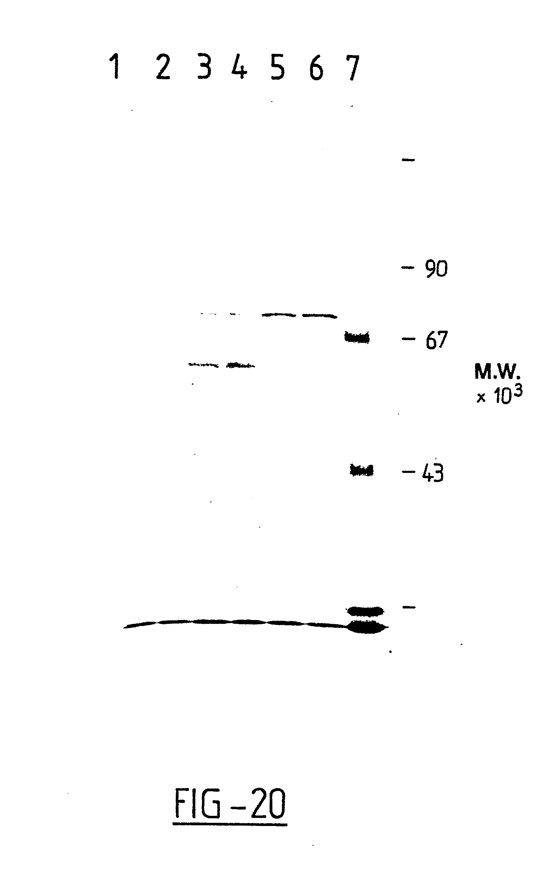

Légendes des figures 19 et 20 :

ligne 1: cellule 3T3,

ligne 2: cellule 3T3 transformée par pMOP,

lignes 3, 4, 5 et 6 :3T3.317.7 concentration de méthotrexate (microgramme/ml).

ligne 1: O

lignes 2 et 3: 0,4,

lignes 4, 5 et 6: 2, 4 et 8 µg respectivement.

Comme cela ressort de la figure 19, en augmentant
la concentration de méthotrexate, on augmente de façon
significative la synthèse de protéine de poids
moléculaire     72 000 daltons. Ces protéines correspondent en première approximation à la protéine de
62 000 daltons qui est immunoprécipitée avec un anti-sérum
spécifique et dans le second cas à la protéine de 72 000
daltons que l'on observe dans l'extrait cellulaire total.

L'extrait cellulaire précédent est analysé
par immunoprécipitation avec un anti-sérum de lapin
anti-facteur IX natif. Les résultats sont reproduits à
la figure 20. Comme on peut le constater, la protéine
de 72 000 daltons qui a été observée à la figure 19
augmente en intensité lorsque l'on augmente la concentration de méthotrexate et elle est immunoprécipitée
spécifiquement avec l'anti-sérum de lapin anti-facteur IX
natif. La relation entre dose et effet pour la protéine
de 62 000 daltons est moins claire. Si comme cela a été
indiqué précédemment, la protéine de 72 000 daltons
représente un précurseur de la protéine de 62 000 daltons,
il est possible que l'impossibilité d'observer une relation
dose-effet pour la protéine de 62 000 daltons soit due
aux conditions expérimentales ; par exemple la durée du marquage est trop courte par rapport au temps nécessaire pour la transformation de la protéine de 72 000 daltons en protéine de 62 000 daltons.

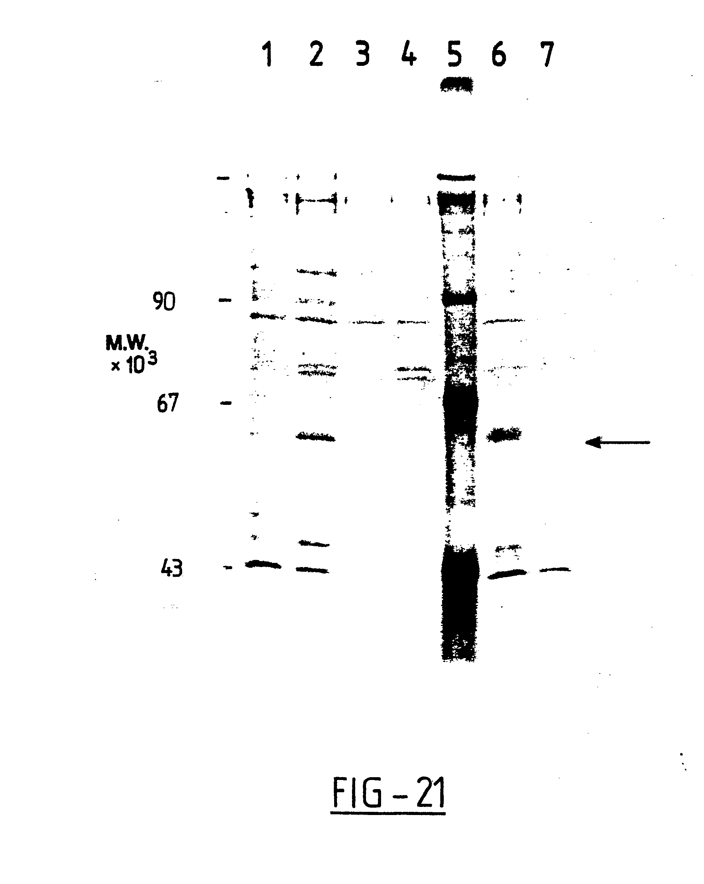

Dans les expériences de la figure 21, les immuno-précipitations sont effectuées en présence ou en l'absence de 10 µg de facteur IX sous forme d'immunocompétiteur. Le résultat démontre que la protéine de 62 000 daltons est spécifiquement immunocompétitive avec le facteur IX natif. Une compétition légère de la protéine de 72 000 daltons est observée, mais les résultats sont beaucoup moins nets.

Légendes de la figure 21 :

ligne 1: extrait de cellule LMTK,

ligne 2: extrait de cellule LMTK co-transformée avec pTK et pTG317,

ligne 3: cellule 3T3

ligne 4: cellule 3T3 transformée par un plasmide,

ligne 5: marqueur de poids moléculaire,

lignes 6 et 7: 3T3.317.7,

Les lignes 1 à 4, 6 et 7 sont immunoprécipitées avec un anti-sérum de lapin anti-facteur IX natif. L'immunoprécipitation dans la ligne 7 est effectuée en présence de 10 µg de facteur IX natif comme compétiteur. La flèche indique la position de la protéine de 62 000 daltons.

De façon à déterminer si la protéine de 62 000 daltons est une glycoprotéine comme cela est suggéré par une nature diffuse sur gel de polyacrylamide SDS et par sa fixation par la Sépharose ConA, les cellules sont mises en incubation pendant une nuit en présence de 3H-mannose, 3H-galactose et 3H-glucosamine. L'incorporation de ces précurseurs radioactifs dans une protéine constitue une indication de l'attachement d'une chaîne de carbohydrate. Les protéines marquées sont alors analysées par immunoprécipitation avec un anti-sérum de lapin anti-facteur IX natif.

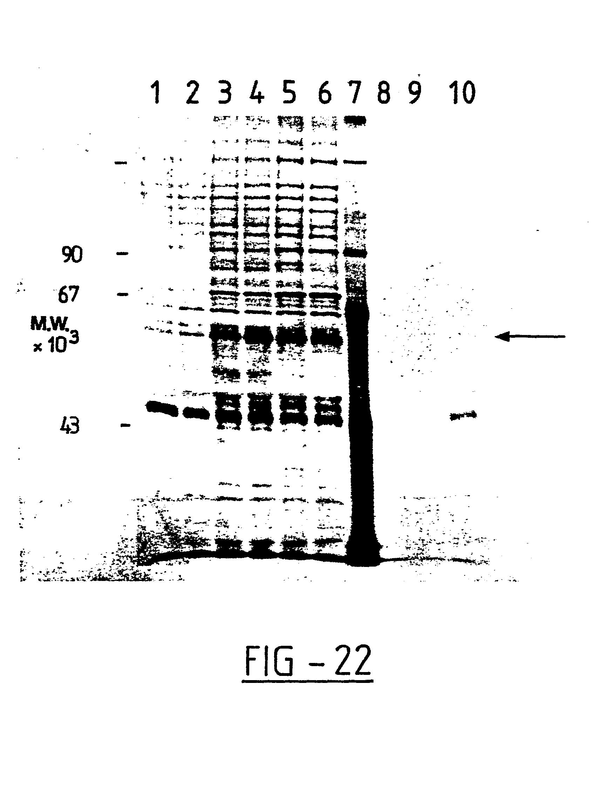

Comme cela ressort de la figure 22, la protéine de 62 000 daltons est marquée dans ces conditions, ce qui confirme la présence d'une chaîne de carbohydrate attachée.

La légende de la figure 22 est la suivante:

lignes 1 à 7: comme pour la figure 20,

lignes 8, 9 et 10: cellule marquée avec 3H-mannose, 3H-galactose et 3H-glucosamine.

ligne 8: cellule 3T3,

ligne 9: cellule 3T3 transformée avec pMOP,

ligne 10: 3T3.317.1.

Conclusions, le facteur IX a été exprimé dans E.coli, Saccharomyces cerevisiae et les lignées cellulaires 3T3 et LMTK. Dans E. coli la taille de la protéine du facteur IX produite (47 000 daltons) correspond à celle du polypeptide mature moins la séquence signal et la pré-protéine.

La détermination de la séquence d'amino-acides de l'extrémité N terminale de la protéine produite bacté-riennement peut être utilisée pour déterminer si tel est bien le cas.

Compte tenu des modifications post-traduction- nelles spécifiques nécessaires pour l'activité du facteur IX, il convient d'envisager la modification du produit bactérien de façon à pouvoir au moins le carboxyler, par exemple en utilisant des préparations de membranes microsomales eucaryotiques (par exemple de foie, de rate ou de rein).

Dans les levures, la taille du produit de facteur IX (57 000 daltons) est nettement plus grande que celui produit dans E. coli. Ceci peut être dû à la glycosylation du facteur IX dans les levures.

Dans les cellules eucaryotiques, deux protéines à 62 000 daltons et à 72 000 daltons immunoprécipitent spécifiquement avec un anti-sérum de lapin anti-facteur IX natif. Une relation de type précurseur-produit peut exister entre ces protéines.

La nature diffuse de la protéine de 62 000 daltons sur gel de polyacrylamide SDN, sa fixation sur la sépharose ConA et l'incorporation de précurseurs radioactifs qui marquent les glycoprotéines dans ces protéines, indiquent largement que la protéine de 62 000 daltons est la forme glycosylée du facteur IX.

Pour une activité biologique, 12 substituants d'acide glutamique à l'extrémité N terminale du facteur IX doivent être 8-carboxylés. Le système enzymatique qui produit cette modification est délicat à manipuler et l'on espère que les lignées cellulaires qui exprimeront couramment le facteur IX effectueront elles-mêmes cette modification.

Une étude préliminaire qui démontre que 100 % de l'antigène de facteur IX présent dans la lignée 3T3.317.1 absorbe le citrate de baryum (une propriété physico-chimique des facteurs de coagulation dépendant de la vitamine K dont on pense qu'elle est en partie due à la présence d'acide $\gamma$-carboxyglutamique), indique que la modification correcte intervient effectivement.

Les souches suivantes ont été déposées à la Collection Nationale de Cultures de Microorganismes (CNCM) de l'Institut Pasteur, 28 rue du Docteur-Roux, Paris 15ème le 30 avril 1985 :

E. coli comportant le plasmide pTG320, sous le n° I-444

E. coli comportant le plasmide pTG318, sous le n° I-443

E. coli comportant le plasmide pTG325, sous le n° I-445

TABLEAU I

| Echantillon | Plasmide | Antigène FIX-Ag (%) | Activité FIX:C (%) |
|---|---|---|---|
| Levure contrôle cellule homog. | pTG834 | 0,0064 | 0,05 |
| Levure FIX cellule homog. | pTG318 | 0,238 | 0,05 |
| Levure contrôle culture sup. | pTG834 | 0,0098 | 0,022 |
| Levure FIX culture sup. | pTG318 | 0,0061 | 0,02 |
| E. coli contrôle homog. 30° | pTG951 | 0,0405 | 0,02 |
| E. coli contrôle homog. 37° | pTG951 | 0,0422 | 0,02 |
| E. coli FIX homog. 30° | pTG320 | 0,328 | 0,08 |
| E. coli FIX homog. 37° | pTG320 | 1,37 | 0,01 |
| 3T3 contrôle culture sup. | – | 0,010 | 9,5 |
| 3T3-317 culture sup. | pTG317 | 0,256 | 9,1 |
| 3T3-317 clone 7 | pTG317 | 0,036 | 10,7 |
| 3T3-317 clone 1 | pTG317 | 0,255 | 10,0 |
| 3T3-317 clone 9 | pTG317 | 0,010 | 8,8 |
| 3T3-317 clone 4 | pTG317 | 0,0743 | 9,4 |
| 3T3 contrôle culture sup. | – | 0,008 | 5,7 |
| 3T3-317-1 | pTG317 | 0,45 | 5,0 |
| 3T3 contrôle culture homog. | – | 0,004 | 12,5 |
| 3T3-317-1 | pTG317 | 1,4 | 0,3 |
| 3T3 contrôle culture $BaCl_2$ ppt. | – | 0,0075 | 3,15 |
| 3T3-317-1 culture $BaCl_2$ ppt. | pTG317 | 1,4 | 1,7 |
| 3T3 contrôle culture sup. précipité avec $BaCl_2$ | – | 0,005 | 0,4 |
| 3T3-317-1 culture sup. précipité avec $BaCl_2$ | pTG317 | 0,09 | 26 |
| 3T3 contrôle culture homog. | – | 0,017 | 30,26 |
| 3T3-317-1 | pTG317 | 1,41 | 0,4 |
| 3T3 contrôle culture sup. | – | 0,027 | 6,5 |
| 3T3-317-1 culture sup. | pTG317 | 0,37 | 7 |
| 3T3-325 clone 1 culture sup. | pTG325 | 0,269 | 8 |
| 3T3-325 clone 2 culture sup. | pTG325 | 0,213 | 8 |
| 3T3-325 clone 3 culture sup. | pTG325 | 0,202 | 10 |
| 3T3-325 clone 4 culture sup. | pTG325 | 0,115 | 9,5 |
| 3T3-325 clone 5 culture sup. | pTG325 | 0,125 | 0,65 |

**0167420**

## BIBLIOGRAPHIE

BERTINA R.M. et VELTKAMP J.J. (1981). In Hemostasis and Thrombosis, Bloom A.L. et Thomas D.T. Eds. pp. 98-110, Churchill Livingstone, London.

HEDNER U. et DAVIE E.W. (1982). in Hemostasis and Thrombosis, Colman R.W., Hirsh J., Marder V.J. et Salzman E.W., Eds. pp. 29-38, J.B. Lippincott Co., Philadelphia.

CHOO K.H., GOULD K.G., REES D.J.G. et BROWNLEE G.G. (1982). Nature 299, 178-180.

KURACHI K. et DAVIE E.W. (1982). Proc. Natl. Acad. Sci. U.S.A. 79, 6461-6464.

TOLSTOSHEV P., BERG R.A., RENNARD S.I., BRADLEY K.N., TRAPNEL B.C. et CRYSTAL R.G. (1981). J.Biol. Chem. 256, 3135-3140.

DENG G. et WU R. (1981). Nucl. Acids Res. 9, 4173-4188.

MURRAY N.E., BRAMMAR W.J. et MURRAY K. (1977). Mol. Gen. Genetics 150, 53-61.

MAcGILLIVRAY R.T.A., DEGEN S.H.F., CHANDRA T., WOO S.L.C. et DAVIE E.W. (1980). Proc. Natl. Acad. Sci. U.S.A. 77, 5153-5157.

CHUNG D.W., RIXON M.W., MAcGILLIVRAY R.T.A. et DAVIE E.W. (1981). Proc. Natl. Acad. Sci. U.S.A. 78, 1466-1470.

KOHLI V., BALLAND A., WINTZERITH M., SAUERWALD R., STAUB A. et LECOCQ J.P. (1982). Nucl. Acids Res. 10, 7439-7448.

FRITZ H.J., BELAGAJE R., BROWN E.L., FRITZ R.H., JONES R.A., LEES R.F. et KHORANA H.G. (1978). Biochemistry 17, 1257-1267.

GRUNSTEIN M. et WALLIS J. (1979). Methods in Enzymology 68, 379-389.

KATAYAMA K., ERICSSON L.H., ENFILED D.L., WALSH K.A., NEURATH H., DAVIE E.W. et TITANI K. (1979). Proc. Natl. Acad. Sci. U.S.A. 76, 4990-4994.

DI SCIPIO R.G., HERMODSON M.A., YATES S.G. et DAVIE E.W. (1977). Biochemistry 16, 698-706.

MESSING J. et VIEIRA J. (1982). Gene 19, 269-276.

　　　　0167420

SANGER F., NICKLEN S. et COULSON A.R. (1977). Proc. Natl. Acad. Sci. U.S.A. 74, 5463-5467.

MAXAM A.M. et GILBERT W. (1980). Methods in Enzymology 65, 499-555.

DI SCIPIO R.G., KURACHI K. et DAVIE E.W. (1978). J. Clin. Inves. 61, 1528-1538.

COOPER D.W. (1978) in The Biochemical Genetics of Man, Brock D.J.H. et MAYO O., pp. 271-324, Eds. Academic Press London.

MIZUOCHI T., TANIGUCHI T., FUJIKAWA K., TITANI K. et KOBATA A. (1983). J. Biol. Chem. 258, 6020-6024.

ISH-HOROWITZ D. et BURKE J.F. (1981). Nucl. Acids Res. 9, 2989-2998.

PANAYOTATOS N. et TRONG K. (1981). Nucl. Acids Res. 9, 5679-5688.

HOOPES B.C. et McCLURE R.R. (1981). Nucl. Acids Res. 9, 5493-5504.

DAGERT M. et EHRLICH S.D., Gene, 23-28 (1979).

VIEIRA J. et MESSING J. Gene 19, 259-268.

ITO M., FUKUDA Y., MURATA K., KIMURA A. (1983). J. Bacteriol. 153, 1, 163-168.

WIGLER et Coll., Cell, vol. 14, 725-731 (1978).

## REVENDICATIONS

1) Vecteur de clonage et d'expression d'une protéine analogue au facteur IX dans des cellules, caractérisé en ce qu'il comporte au moins :

- une séquence d'ADN codant pour ladite protéine analogue au facteur IX : ADN.FIX ;

- les éléments assurant l'expression de cette séquence dans lesdites cellules.

2) Vecteur selon la revendication 1, caractérisé en ce qu'il comporte une origine de réplication autonome dans lesdites cellules.

3) Vecteur selon l'une des revendications 1 et 2, caractérisé en ce qu'il comporte un caractère de sélection positif s'exprimant dans lesdites cellules.

4) Vecteur selon l'une des revendications 1 à 3, caractérisé en ce qu'il comporte en amont de la séquence d'ADN.FIX au moins un promoteur.

5) Vecteur selon la revendication 4, caractérisé en ce que la promotion de la séquence d'ADN.FIX est assurée par un promoteur bactérien suivi d'un site de fixation des ribosomes.

6) Vecteur selon la revendication 5, caractérisé en ce que le promoteur est constitué par tout ou partie du promoteur PL du phage λ.

7) Vecteur selon l'une des revendications 5 et 6, caractérisé en ce que le site de fixation des ribosomes comporte au moins la séquence :

ATAACACAGGAACAGATCT$\overline{\text{ATG}}$

8) Vecteur selon l'une des revendications 3 à 7, caractérisé en ce que le caractère de sélection positif est un caractère de résistance à un antibiotique s'exprimant dans les bactéries.

9) Vecteur selon l'une des revendications 1 à 4, caractérisé en ce que la promotion de la séquence d'ADN.FIX est assurée par un promoteur de levure et en ce que la séquence d'ADN.FIX est terminée par un terminateur de levure.

10) Vecteur selon la revendication 9, caractérisé en ce que le promoteur et le terminateur sont le promoteur et le terminateur de PGK.

11) Vecteur selon l'une des revendications 10 et 11, caractérisé en ce que l'origine de réplication est l'origine de réplication du plasmide 2ψ.

12) Vecteur selon l'une des revendications 9 à 11, caractérisé en ce que le caractère de sélection positif est fourni par le gène URA3.

13) Vecteur selon l'une des revendications 1 à 4, caractérisé en ce qu'il comporte une origine de réplication dans les virus.

14) Vecteur selon la revendication 13, caractérisé en ce qu'il comporte l'origine de réplication de SV40 ou de BPV.

15) Vecteur selon l'une des revendications 13 et 14, caractérisé en ce que le promoteur est constitué de tout ou partie d'un promoteur de SV40 ou par tout ou partie du promoteur de la thymidine kinase du virus Herpes simplex.

16) Vecteur selon l'une des revendications 13 à 15, caractérisé en ce qu'il comporte en outre un gène marqueur s'exprimant dans les cellules eucaryotes.

17) Vecteur selon l'une des revendications 13 à 16, caractérisé en ce que la séquence d'ADN FIX est suivie d'un intron et/ou d'un site de polyadénylation.

18) Vecteur selon l'une des revendications 13 à 17, caractérisé en ce qu'il comporte tout ou partie du gène du BPV et une séquence comportant dans l'ordre tout ou partie d'un promoteur de virus, la séquence d'ADN FIX, un intron et un site de polyadénylation.

19) Vecteur selon l'une des revendications 5 à 8, caractérisé en ce qu'il s'agit du plasmide pTG320.

20) Vecteur selon l'une des revendications 9 à 12, caractérisé en ce qu'il s'agit du plasmide pTG318 ou pTG324.

21) Vecteur selon l'une des revendications 13 à 18, caractérisé en ce qu'il s'agit du plasmide pTG317 ou pTG325.

22) Bactéries transformées par un plasmide selon l'une des revendications 1 à 8 et 19.

23) Bactéries selon la revendication 22, caractérisées en ce qu'il s'agit d'une souche de E. coli.

24) Levures transformées par un plasmide selon l'une des revendications 1 à 4, 9 à 12 et 20.

25) Levures selon la revendication 24, caractérisées en ce qu'il s'agit d'une souche de Saccharomyces cerevisiae.

26) Cellules de mammifères transformées par un vecteur selon l'une des revendications 1 à 4, 13 à 18 et 21.

27) Procédé de préparation d'une protéine analogue au facteur IX, caractérisé en ce qu'on cultive une bactérie, levure ou cellule selon l'une des revendications 22 à 26 et que l'on récupère la protéine analogue au facteur IX produite.

28) Protéine analogue au facteur IX obtenue par la mise en oeuvre du procédé selon la revendication 27.

29) Protéine analogue au facteur IX obtenue au moins en partie par culture de cellules bactériennes, de levures ou de mammifères.

35 40 45 50

a. thr glu lys thr thr glu phe trp lys gln tyr val asp gly asp gln cys glu ser

b. ACX GA$_G^A$ AA$_G^A$ ACX ACX GA$_G^A$ TT$_T^C$ TGG AA$_G^A$ CA$_G^A$ TA$_T^C$ GTX GA$_T^C$ GGX GA$_T^C$ CA$_G^A$ TG$_T^C$ GA$_G^A$ TCX
$_{AG}^{C}$

c. T GAG AAGACT ACA GAG TTC TGG AAG CAG TAT GTG GAT GGT GAT CAG TGT GAG

d. TGAA AGAACA ACTGAA TTT TGGAAG CAG TAT GTT GAT GGAGAT CAGTGT GAG

244 250 260 270 280 290

## FIG.1

FIG. 2

```
1                          25                          50                          75
T ATG CAG CGC GTG AAC ATG ATC ATG GCA GAA TCA CCA GGC CTC ATC ACC ATC TGC CTT TTA GGA TAT CTA CTC AGT
  met gln arg val asn met ile met ala glu ser pro gly leu ile thr ile cys leu leu gly tyr leu leu ser
                           100                         125                         150
GCT GAA TGT ACA GTT TTT CTT GAT CAT GAA AAC GCC AAC AAA ATT CTG AAT CGG CCA AAG AGG TAT AAT TCA GGT
ala glu cys thr val phe leu asp his glu asn ala asn lys ile leu asn arg pro lys arg tyr asn ser gly
                           175                         200                         225
AAA TTG GAA GAG TTT GTT CAA GGG AAC CTT GAG AGA GAA TGT ATG GAA GAA AAG TGT AGT TTT GAA GAA GCA CGA
lys leu glu glu phe val gln gly asn leu glu arg glu cys met glu glu lys cys ser phe glu glu ala arg
                           250                         275                         300
GAA GTT TTT GAA AAC ACT GAA AGA ACA ACT GAA TTT TGG AAG CAG TAT GTT GAT GGA GAT CAG TGT GAG TCC AAT
glu val phe glu asn thr glu arg thr thr glu phe trp lys gln tyr val asp gly asp gln cys glu ser asn
                           325                         350                         375
CCA TGT TTA AAT GGC GGC AGT TGC AAG GAT GAC ATT AAT TCC TAT GAA TGT TGG TGT CCC TTT GGA TTT GAA GGA
pro cys leu asn gly gly ser cys lys asp asp ile asn ser tyr glu cys trp cys pro phe gly phe glu gly
                           400                         425                         450
AAG AAC TGT GAA TTA GAT GTA ACA TGT AAC ATT AAG AAT GGC AGA TGC GAG CAG TTT TGT AAA AAT AGT GCT GAT
lys asn cys glu leu asp val thr cys asn ile lys asn gly arg cys glu gln phe cys lys asn ser ala asp
                           475                         500                         525
AAC AAG GTG GTT TGC TCC TGT ACT GAG GGA TAT CGA CTT GCA GAA AAC CAG AAG TCC TGT GAA CCA GCA GTG CCA
asn lys val val cys ser cys thr glu gly tyr arg leu ala glu asn gln lys ser cys glu pro ala val pro
                           550                         575                         600
TTT CCA TGT GGA AGA GTT TCT GTT TCA CAA ACT TCT AAG CTC ACC CGT GCT GAG ACT GTT TTT CCT GAT GTG GAC
phe pro cys gly arg val ser val ser gln thr ser lys leu thr arg ala glu thr val phe pro asp val asp
                           625                         650                         675
TAT GTA AAT TCT ACT GAA GCT GAA ACC ATT TTG GAT AAC ATC ACT CAA AGC ACC CAA TCA TTT AAT GAC TTC ACT
tyr val asn ser thr glu ala glu thr ile leu asp asn ile thr gln ser thr gln ser phe asn asp phe thr
                           700                         725                         750
CGG GTT GTT GGT GGA GAA GAT GCC AAA CCA GGT CAA TTC CCT TGG CAG GTT GTT TTG AAT GGT AAA GTT GAT GCA
arg val val gly gly glu asp ala lys pro gly gln phe pro trp gln val val leu asn gly lys val asp ala
                           775                         800                         825
TTC TGT GGA GGC TCT ATC GTT AAT GAA AAA TGG ATT GTA ACT GCT GCC CAC TGT GTT GAA ACT GGT GTT AAA ATT
phe cys gly gly ser ile val asn glu lys trp ile val thr ala ala his cys val glu thr gly val lys ile
                           850                         875                         900
ACA GTT GTC GCA GGT GAA CAT AAT ATT GAG GAG ACA GAA CAT ACA GAG CAA AAG CGA AAT GTG ATT CGA ATT ATT
thr val val ala gly glu his asn ile glu glu thr glu his thr glu gln lys arg asn val ile arg ile ile
                           925                         950                         975
CCT CAC CAC AAC TAC AAT GCA GCT ATT AAT AAG TAC AAC CAT GAC ATT GCC CTT CTG GAA CTG GAC GAA CCC TTA
pro his his asn tyr asn ala ala ile asn lys tyr asn his asp ile ala leu leu glu leu asp glu pro leu
                           1000                        1025                        1050
GTG CTA AAC AGC TAC GTT ACA CCT ATT TGC ATT GCT GAC AAG GAA TAC ACG AAC ATC TTC CTC AAA TTT GGA TCT
val leu asn ser tyr val thr pro ile cys ile ala asp lys glu tyr thr asn ile phe leu lys phe gly ser
                           1075                        1100                        1125
GGC TAT GTA AGT GGC TGG GGA AGA GTC TTC CAC AAA GGG AGA TCA GCT TTA GTT CTT CAG TAC CTT AGA GTT CCA
gly tyr val ser gly trp gly arg val phe his lys gly arg ser ala leu val leu gln tyr leu arg val pro
                           1150                        1175                        1200
CTT GTT GAC CGA GCC ACA TGT CTT CGA TCT ACA AAG TTC ACC ATC TAT AAC AAC ATG TTC TGT GCT GGC TTC CAT
leu val asp arg ala thr cys leu arg ser thr lys phe thr ile tyr asn asn met phe cys ala gly phe his
                           1225                        1250                        1275
GAA GGA GGT AGA GAT TCA TGT CAA GGA GAT AGT GGG GGA CCC CAT GTT ACT GAA GTG GAA GGG ACC AGT TTC TTA
glu gly gly arg asp ser cys gln gly asp ser gly gly pro his val thr glu val glu gly thr ser phe leu
                           1300                        1325                        1350
ACT GGA ATT ATT AGC TGG GGT GAA GAG TGT GCA ATG AAA GGC AAA TAT GGA ATA TAT ACC AAG GTA TCC CGG TAT
thr gly ile ile ser trp gly glu glu cys ala met lys gly lys tyr gly ile tyr thr lys val ser arg tyr
                           1375                        1400                        1425
GTC AAC TGG ATT AAG GAA AAA ACA AAG CTC ACT TAA TGA AAG ATG GAT TTC CAA GGT TAA TTC ATT GGA ATT GAA
val asn trp ile lys glu lys thr lys leu thr --- ---
                           1450                        1475                        1500
AAT TAA CAG GGC CTC TCA CTA ACT AAT CAC TTT CCC ATC TTT TGT TAG ATT TGA ATA TAT ACA TTC TAT GAT CAT
                           1525                        1550                        1575
TGC TTT TTC TCT TTA CAG GGG AGA ATT TCA TAT TTT ACC TGA GCA AAT TGA TTA GAA AAT GGA ACC ACT AGA GGA
                           1600                        1625
ATA TAA TGT GTT AGG AAA TTA CAG TCA TTT CTA AGG GCC CAG CCT TGA CAA ATT GTG AGT AAA ...............
```

FIG - 3

met--- --- --- --- --- ser ala--- --- --- --- lysargtyr--- --- arg ala --- arg val --- ser --- --- thr
-46       -21     $\delta_{12}$    145    180    415

CHO
CHO

S—S

FIG_4

FIG_5

## FIG.6

0167420

FIG.7

FIG_8

FIG_9

**0167420**

## CONSTRUCTION DE PTG909

c II    loc Z')

pTG908

P_L N    S/D  ATG

N de I    BamHI , Hind III : Pst I

N de I

Eco RII

Sau 3A

Adaptateur
synthétique

IFN - γ

TATGTGTTATTG
ACACAATAACGGTCC

IFN - γ    lac Z')

P_L N    S/D    ATG

S/D    fmet cys tyr cys gln asp pro
TCTAAGGAAGTACTTACATATGTGTTATTGCCAGGACCCA
AGATTCCTTCATGAATGTATACACAATAACGGTCCTGGGT

FIG_10

0167420

## CONSTRUCTION DE PTG941

PTG909

IFN

*MetCysTyrCysGlnAspProTyr*
TAAGGAAGTACTTACATATGTGTTATTGCCAGGACCCATATG....
                    NDEI                    NDEI

NDEI

+

TATGTGCTACTGTCAGGATCCC
ACACGATGACAGTCCTAGGGAT

IFN

*MetCysTyrCysGlnAspProTyr*
PTG941
TAAGGAAGTACTTACATATGTGCTACTGTCAGGATCCCTAT
                   NDEI              BAMHI

## FIG. 11

FIG_12

FIG.13

FIG_14

FIG_15

0167420

6 5 4 3 2 1

−67

−43

MW
x 10³

−25,7

FIG − 16

FIG -17

0167420

FIG -18

FIG -19

1  2  3  4  5  6  7

– 90

– 67

M.W.
× 10³

– 43

FIG –20

FIG – 21

FIG – 22

Office européen
des brevets

**RAPPORT DE RECHERCHE EUROPEENNE**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| Y | NUCLEIC ACIDS RESEARCH, vol. 11, no. 8, avril 1983, pages 2325-2335, IRL Press Ltd., Oxford, GB; M. JAYE et al.: "Isolation of a human anti-haemophilic factor IX cDNA clone using a unique 52-base synthetic oligonucleotide probe deducted from the amino acid sequence of bovine factor IX" * En entier * | 1-29 | C 12 N 15/00<br>C 12 N 1/20<br>C 12 N 1/18<br>C 12 N 5/00<br>C 12 N 9/50<br>A 61 K 35/16 |
| | --- | | |
| Y | EP-A-0 107 278 (NATIONAL RESEARCH DEVELOPMENT CORP.) * Page 4, ligne 20 - page 5, ligne 10; page 6, ligne 26 - page 7, ligne 24; page 25, ligne 34 - page 28, ligne 32; revendications * | 1-29 | |
| | --- | | **DOMAINES TECHNIQUES RECHERCHES (Int. Cl 4)** |
| Y | EP-A-0 094 887 (TRANSGENE S.A.) * Page 5, ligne 30 - page 10, ligne 34; page 27, ligne 1 - page 33, ligne 7; revendictions 1,8-23 * | 1-8,19,22,23,27-29 | C 12 N<br>C 12 P<br>A 61 K |
| | --- | | |
| Y | EP-A-0 073 635 (A.J. KINGSMAN et al.) * Page 4, ligne 10 - page 5, ligne 32; page 16, ligne 1 - page 19, ligne 25; revendications 1-4,8,10 * | 1-4,8-11 | |
| | ---    -/- | | |

Le présent rapport de recherche a été établi pour toutes les revendications

| Lieu de la recherche | Date d'achèvement de la recherche | Examinateur |
|---|---|---|
| LA HAYE | 09-07-1985 | DESCAMPS J.A. |

## Office européen des brevets

## RAPPORT DE RECHERCHE EUROPEENNE

| DOCUMENTS CONSIDERES COMME PERTINENTS | | | |
|---|---|---|---|
| Catégorie | Citation du document avec indication. en cas de besoin des parties pertinentes | Revendication concernée | CLASSEMENT DE LA DEMANDE (Int Cl 4) |
| Y | EP-A-0 001 562 (ANVAR)<br>* Page 3, lignes 13-18; revendication 1 *<br>--- | 12 | |
| Y | EP-A-0 073 656 (GENETECH, INC.)<br><br>* Page 11, ligne 11 - page 12, ligne 13; page 12, ligne 31 - page 13, ligne 19; page 19, ligne 13 - page 22, ligne 14; page 24, ligne 25 - page 26, ligne 23; revendications 1,4,5 *<br>--- | 1-4,8, 13-16, 21,26-29 | |
| Y | US-A-4 419 446 (P.M. HOWLEY)<br>* En entier *<br>--- | 14,18 | |
| Y | GENE, vol. 24, 1983, pages 281-287, Elsevier Science Publishers; J.H. LUPKER et al.: "Abundant excretion of human growth hormone by recombinant-plasmid-transformed monkey kidney cells"<br>* En entier *<br>---     -/- | 17-18 | DOMAINES TECHNIQUES RECHERCHES (Int Cl.4) |

Le présent rapport de recherche a ete établi pour toutes les revendications

| Lieu de la recherche | Date d'achèvement de la recherche | Examinateur |
|---|---|---|
| LA HAYE | 09-07-1985 | DESCAMPS J.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

| | DOCUMENTS CONSIDERES COMME PERTINENTS | | Page 3 |
|---|---|---|---|
| Catégorie | Citation du document avec indication, en cas de besoin, des parties pertinentes | Revendication concernee | CLASSEMENT DE LA DEMANDE (Int Cl 4) |
| Y | NUCLEID ACIDS RESEARCH, vol. 11, no. 20, octobre 1983, pages 7119-7136, IRL Press Ltd., Oxford, GB; R. BREATHNACH et al.: "Plasmids for the cloning and expression of full-length double-stranded cDNAs under control of the SV40 early or late gene promoter" * En entier * | 17-18 | |
| Y | WO-A-8 203 087 (INSTITUT PASTEUR) * Revendications 1,5 * | 15 | |
| X | CHEMICAL ABSTRACTS, vol. 86, no. 7, 14 février 1977, page 314, no. 40873u, Columbus, Ohio, US; H. SUOMELA: "Human coagulation actor IX. isolation and characterization" & EUR. J. BIOCHEM. 1976, 71(1), 145-54 * Résumé * | 28,29 | |
| | | | DOMAINES TECHNIQUES RECHERCHES (Int. Cl 4) |

Le present rapport de recherche a ete établi pour toutes les revendications

| Lieu de la recherche | Date d'achèvement de la recherche | Examinateur |
|---|---|---|
| LA HAYE | 09-07-1985 | DESCAMPS J.A. |